# EUROPEAN PATENT SPECIFICATION

(11) **EP 3 371 595 B1**
(45) Date of publication and mention of the grant of the patent: **25.12.2019**
(21) Application number: 16801976.8
(22) Date of filing: 04.11.2016
(51) Int. Cl.: G01N 33/50

(54) **EX VIVO METHOD FOR TESTING CELLULAR RESPONSIVENESS OF PRIMARY TUMOR CELL POPULATIONS TO A DRUG OR COMBINATION OF DRUGS**
EX VIVO-VERFAHREN ZUR PRÜFUNG DER ZELLULAREN ANTWORT VON PRIMÄRZELLENPOPULATIONEN AUF EINE DROGE ODER KOMBINATIONEN VON DROGEN
PROCÉDÉ EX VIVO POUR TESTER LA RÉPONSE CELLULAIRE DE POPULATIONS DE CELLULES PRIMAIRES À UN MÉDICAMENT OU À UNE COMBINAISON DE MÉDICAMENTS

(30) Priority: 04.11.2015 US 201562250687 P
(43) Date of publication of application: 12.09.2018
(73) Proprietor: Vivia Biotech, S.L., 28760 Madrid (ES)
(72) Inventor: BALLESTEROS NOBELL, Juan Antonio, 28760 Madrid (ES); BENNETT, Teresa, 28760 Madrid (ES); PRIMO RAMOS, Daniel, 28760 Madrid (ES); PROSPERO GHIA, Paolo, 20132 Milano (IT); ESPINOSA OQUILLAS, Ana Belén, 28760 Madrid (ES); GORROCHATEGUI GUILLÉN, Julián, 28760 Madrid (ES); ROBLES MATEOS, Alicia, 28760 Madrid (ES); HERNÁNDEZ CAMPO, Pilar, 28760 Madrid (ES)
(74) Representative: Manuel Illescas y Asociados, S.L.U.
(86) International application number: PCT/EP2016/076690
(87) International publication number: WO 2017/077046

(56) References cited:
- WO-A1-2010/120329
- WO-A1-2014/035668
- WO-A1-2015/042450
- US-A1- 2010 298 255
- BALLESTEROS J. ET AL.: "An innovative high-throughput ex vivo drug assay incorporating the native microenvironment reveals a novel mechanism of action of idelalisib in CLL", BLOOD, vol. 126, no. 23, 2485, 2015, pages 1-6, XP009192990,
- BENNETT T.A. ET AL.: "Pharmacological profiles of acute myeloid leukemia treatments in patient samples by automated flow cytometry: A bridge to individualized medicine", CLIN. LYMPHOMA MYELOMA LEUK., vol. 14, no. 4, August 2014 (2014-08), pages 305-318, XP55333568, cited in the application

## Description

### FIELD OF THE INVENTION

Present invention relates to the field of pharmacology and, in particular, to the assessment of anti-tumor drugs activity.

### BACKGROUND OF THE INVENTION

The microenvironment (ME) critically affects tumor cell survival, proliferation and drug resistance (1). The role of the ME has become even more important with the discovery and development of cancer drugs that specifically target components of the ME (2). This has become an obstacle for the *ex vivo* testing of drugs that treat cancer. The absence of the factors that constitute the ME in these *ex vivo* assays will influence the perceived efficacy of many drugs. The result of this is a lack of correlation between the *ex vivo* results and the clinical effectiveness of these drugs.

Several groups have developed microenvironment-mimicking assays, to varying degrees of success, to try and overcome some of these obstacles. Those methods, disclosed in the prior art, include adding cellular elements (*3-5*), adding components of the extracellular matrix (6, 7) and/or the addition of cocktails of soluble cytokines/chemokines or molecules derived from non-tumor cells present in the tumor microenvironment (*8, 9*). WO 2014/035668 A1 describes an assay for detecting malignant potential, involving *in vitro* co-culturing a population of cancer responder cells (e.g., primary cancer/tumor cells) with a population of non-tumor cells (e.g., bone marrow-derived circulating cells, buffy coat cells, peripheral circulating cells, and immune cells), contacting the co-culture with at least one drug, and measuring at least one malignant phenotype exhibited by the cancer responder cells, such as ability to proliferation on soft agar, ability to proliferate and form tumor in vitro, ability to proliferate and form tumor in vivo. The method may further comprise co-culturing the populations of cancer responder cells and non- tumor cells in the presence of plasma. The subject may have been diagnosed with cancer and the plasma may be obtained from said subject. The subject may have undergone chemotherapy. The method may be used for determining the likelihood of development of cancer drug resistance in a subject. The cancer responder cells may be human tumor cell lines, fresh or frozen human tumor surgical samples, fresh or frozen human biopsy samples, human tumor cells. WO 2015/042450 A1 discloses systems and methods for studying responses of patient target cells in the presence of their own stroma cells (cis-co-culture) under different drug conditions. WO 2010/120329 A1 discloses extracellular matrix bioscaffolds capable of supporting the formation and growth of tumors from tumor cells surrounded by tumor-associated cells such as macrophages and fibroblast-like cells and discloses the importance of tumor microenvironment in methods for testing compounds for treating cancers. While these attempts have had some success, the present invention introduces a new method for mimicking components of the microenvironment that have not previously been attempted.

Chronic Lymphocytic Leukemia (CLL) is a prototype of a disease with an extremely heterogeneous biology and clinical course; it is the most frequent adult hematologic malignancy (*10*). This pronounced clinical heterogeneity is linked to both cell-intrinsic and cell-extrinsic mechanisms of the disease. The best known amongst cell-intrinsic mechanisms concerns genomic aberrations, epigenetic modifications and micro-RNAs. CLL behavior and outcome, including chemorefractoriness, also depends on stimuli present in the leukemic ME (*11*). The critical role of external stimulation is shown by the remarkable therapeutic activity of drugs interfering with CLL-ME interactions, namely inhibitors of signaling through the clonotypic B cell receptors (BcR); these novel drugs (Ibrutinib and Idelalisib) constitute a major paradigm shift in CLL treatment (*12, 13*).

Multiple Myeloma (MM) is a malignant disease characterized by proliferation of clonal plasmatic cells in the bone marrow (BM). MM is still an incurable disease, being the second most frequent hematological malignancy in the USA and Europe. MM cells have a strong BM microenvironment dependence, which confers anti-apoptotic and pro-survival signals and resistance to drugs. MM tumor cells have an elevated proliferation rate promoted by soluble factors and molecular interactions within the particular BM microenvironment.

Here we show that the addition of an Artificial Environment (AE) to *ex vivo* assays using isolated primary tumor cells, had a profound effect on viability and proliferation of said tumor cells. This constitutes a novel method for improving the responsiveness to drugs, particularly anti-tumor drugs, of primary tumor cells, *in ex vivo* assays.

Tumor samples extracted from cancer patients contain the tumor cells in the context of a highly heterogeneous environment. This environment may represent 99% of the sample extracted from the patient, for example in hematological tissue samples such as bone marrow, peripheral blood, or lymph node. To study these primary tumor cells, it is common practice to remove most of this heterogeneous environment, concentrating the remaining tumor cells that are being studied. In hematological samples this is commonly performed by a density gradient isolating the fraction containing the leukocytes, which corresponds roughly to only 1% of the entire sample. The other major fractions of the density gradient commonly discarded are the plasma fraction, and the erythrocyte fraction. We refer to both of these discarded fractions as the Artificial Environment (AE). These discarded fractions could be quite relevant to the leukocytes and discarding them may lead to artifacts in the leukocyte's biology. We have investigated the effect of these discarded fractions on the activity of compounds on primary tumor cells, by performing a density gradient isolation and by adding Artificial Environment fractions isolated therein from the same or different patient samples to primary tumors cells.

The disclosed method of the present invention adds Artificial Environment to primary tumor cells. The present invention has several advantages over that of the prior art. The activity of compounds on primary cells is artificially altered in assays which use isolated primary cells *ex vivo.* The method of the present invention, in which the assay of potential anti-tumor drugs is also included, adds an Artificial Environment that compensates for the lack of a microenvironment in isolated cells. In some prior assays, the whole sample has been used, but it is generally greatly diluted. Adding AE to whole sample assays would compensate for artifacts imposed by the initial dilution of the ME of the sample. Another advantage is that the methods of present invention enhance the viability of a primary tumor cell population. An additional advantage is that the method of the present invention promotes the proliferation of a primary tumor cell population, to levels that have not been previously reported (*14*). These advantages allow for the *ex vivo* analysis of the cellular response of the primary tumor cell population to a large number of variables, including different incubation times and testing of many drug compositions, including drugs that inhibit proliferation. Moreover, another advantage of the present invention is the capacity to analyze the primary tumor cells in a manner that more closely mimics the *in vivo* microenvironment of a patient. This is particularly important in the case of drugs targeting the microenvironment e.g. ibrutinib and idelalisib, whose assessment will likely require microenvironment-mimicking assays. Investigating the effect of these compounds and their combinations with other compounds, with the Artificial Environment, prevents the major artifacts actually existing with the assays based on isolated primary tumor cells. Methods of the present invention afford, therefore, the advantage of improving the ability of an *ex vivo* test to determine if a drug or drugs would be beneficial to a patient (or patients).

### DESCRIPTION

### Brief description of the invention

The invention presented here relates to a method for producing an artificial environment (AE) of primary cell populations, particularly an artificial tumor environment of primary tumor cell populations and its use in an *ex vivo* method to test the cellular responsiveness of primary tumor cell populations to a drug or combination of drugs. The test of the cellular responsiveness comprises incubation of the primary tumor cells with the artificial tumor environment and the drug or drugs to be tested and analyze the response of the primary tumor cell populations. The incubation of the primary tumor cells with the artificial tumor environment, enhances the viability of said tumor cells and/or induces greater levels of tumor cell proliferation and, consequently, increases the sensitivity and accuracy of the test with regard to the drug/s assayed.

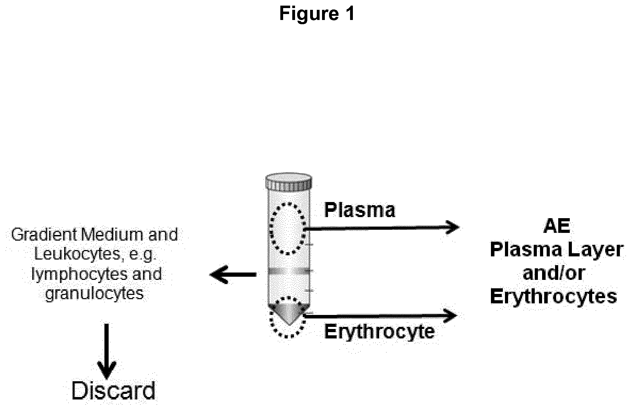

Described herein is also the process for obtaining components of AE from peripheral blood (PB), or bone marrow (BM), or lymph nodes (LN) samples. Said components are used to stabilize primary tumor cell populations. These selected components which constitute the AE are isolated and/or mixed components from a sample selected from: peripheral blood, or bone marrow, or lymph nodes obtained from a donor (Figure 1 and Example 1), but excluding the leukocytes fraction (AE leukocyte-free).

Reference will now be made in detail to the preferred embodiments of the invention, examples of which are provided hereto. Although the disclosure herein refers to certain illustrated embodiments, it is to be understood that these embodiments are presented by way of example and not by way of limitation. The intent of the following detailed description, although discussing exemplary embodiments, is to be construed to cover all modifications, alternatives and equivalents of the embodiments as may fall within the scope of the invention as defined by the appended claims. The disclosed methods may be utilized in conjunction with various medical procedures that are conventionally used in the art.

### Description of the Figures

**Figure 1****.** Method for obtaining the Artificial Environment (AE). The components of a sample of peripheral blood, bone marrow or lymph node are separated into various component parts by density gradient centrifugation. The leukocyte fraction is discarded and the plasma layer and the packed erythrocytes were combined to create the AE.

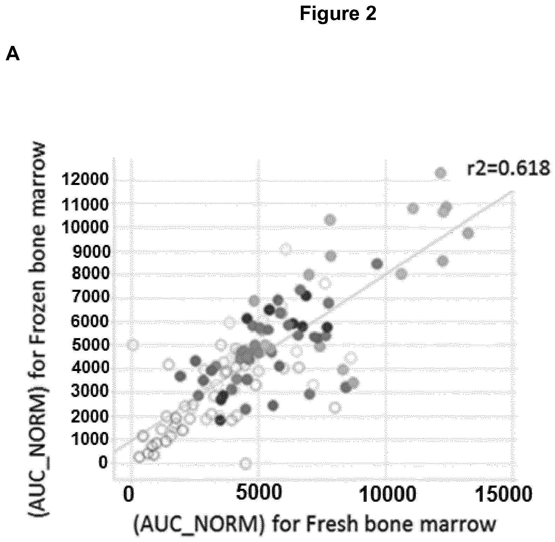

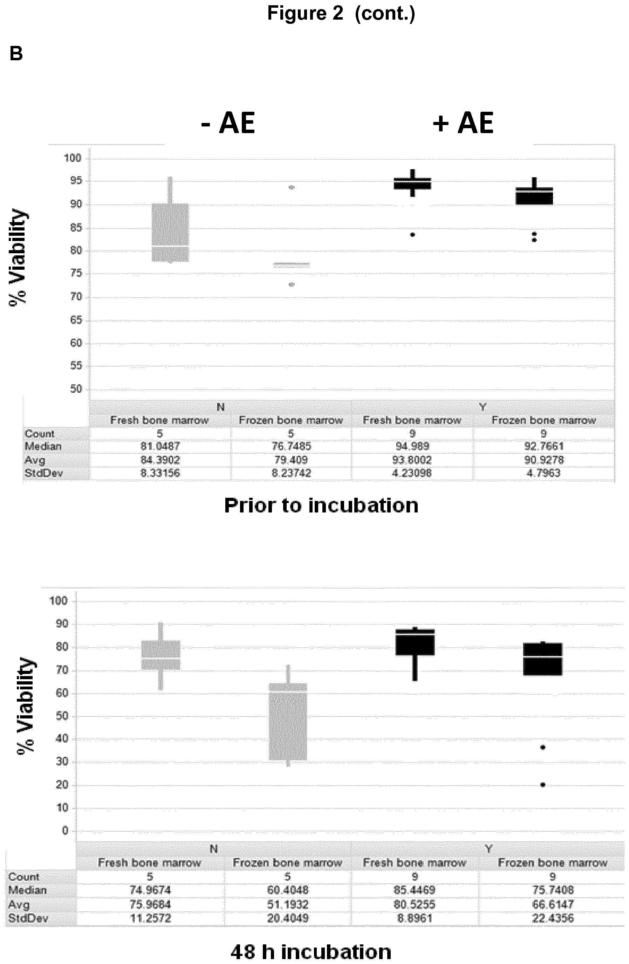

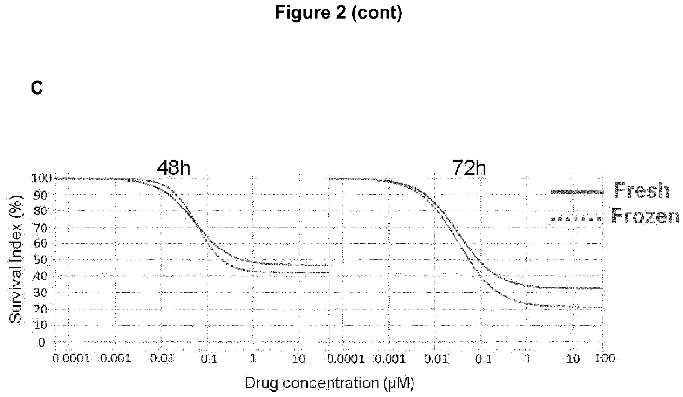

**Figure 2****.** AE enables good correlation of results between fresh and frozen primary tumor cells of AML patients. Figure 2A represents the correlation between the Area Under the Curve (AUC) of fresh and frozen AML samples for 7 different drugs at 24h (filled circles) and at 48h (empty circles). The different color intensity of the filled circles represents different drugs. Figure 2B represents the percentage of viability in fresh and frozen AML bone marrow samples adding or without adding AE at basal level (upper panel) and after 48h incubation (lower panel). Figure 2C shows the Dose-Response curves at 48h (left) and 72h (right) for a drug in an ALL sample. Solid line represents the fresh sample and the dotted line represents the frozen sample reconstituted with AE (plasma and erythrocytes fractions).

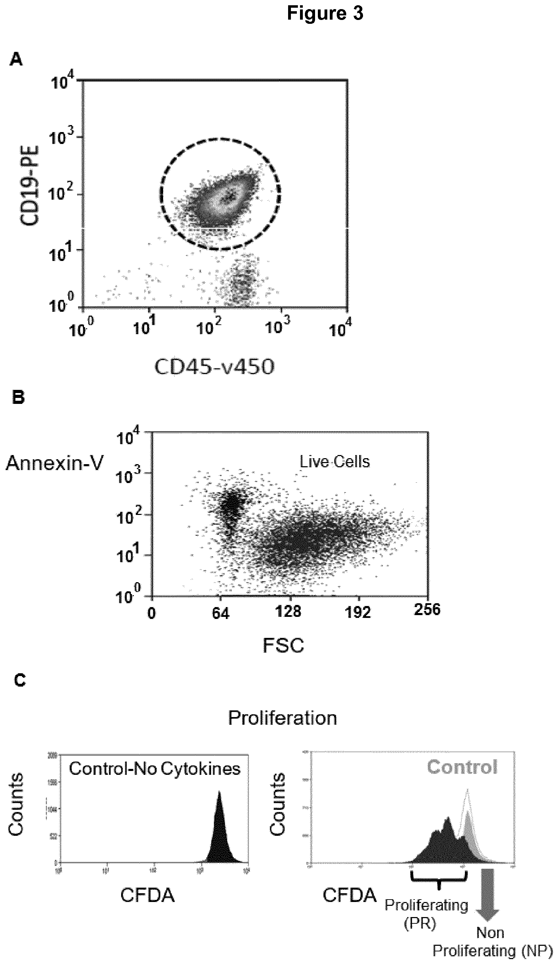

**Figure 3****.** Use of cell surface markers to identify primary tumor cell population. Annexin-V is used to evaluate viability and CFDA to monitor proliferation. Upper panel A represents the gating strategy by flow cytometry to identify B-CLL cells. The middle panel B represents the identification between apoptotic and viable B-CLL cells according to the Annexing-V expression. The lower panels C, right and left represents one example to identify the proliferating population according to the CFDA expression.

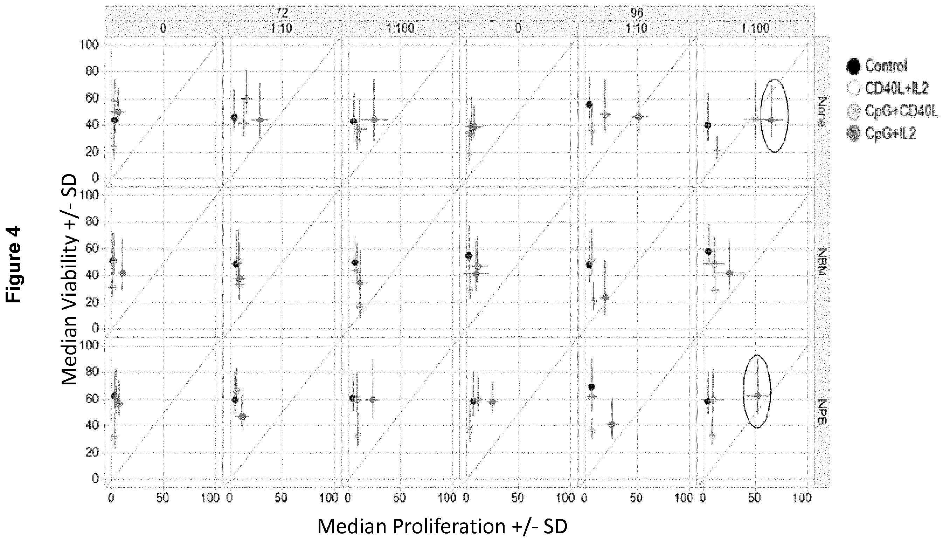

**Figure 4****.** Viability and proliferation with AE derived from NPB and NBM and +/- HS-5 cells to determine best cytokine protocol. The median response is shown for 5 CLL primary tumor samples for each condition tested, incubated with AE from either NPB or NBM, +/- HS-5 cell line (0, 1:10, 1:100), at 72h or 96h. These conditions are tested for three cytokine combinations (sCD40L+IL-2, CpG+sCD40L, CpG+IL-2) plus controls.

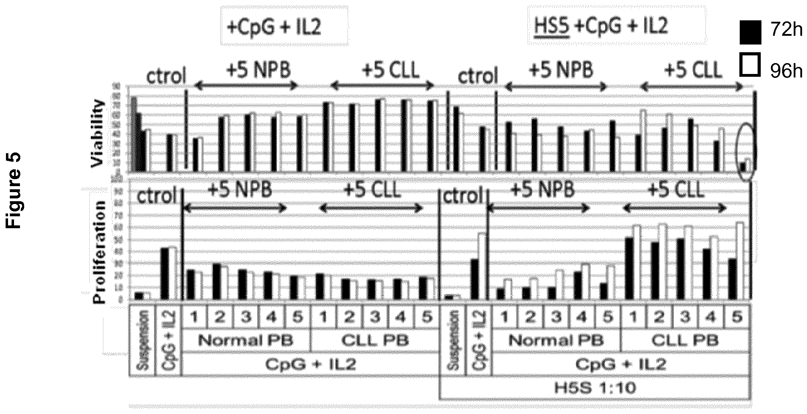

**Figure 5****.** Viability and proliferation with AE derived from NPB and CLLPB using best conditions from Figure 4. AE from 5 donors was tested in a CLL primary tumor sample. Figure 5 represents the percentage of viability (upper panel) or proliferation (bottom panel) of the CLL sample with the addition of CpG+IL2 (left panel) or HS-5+CpG+IL2 (right panel) together with the mixture of the AE. Control samples refer to the CLL primary tumor sample without AE, plus or minus CpG+IL2.

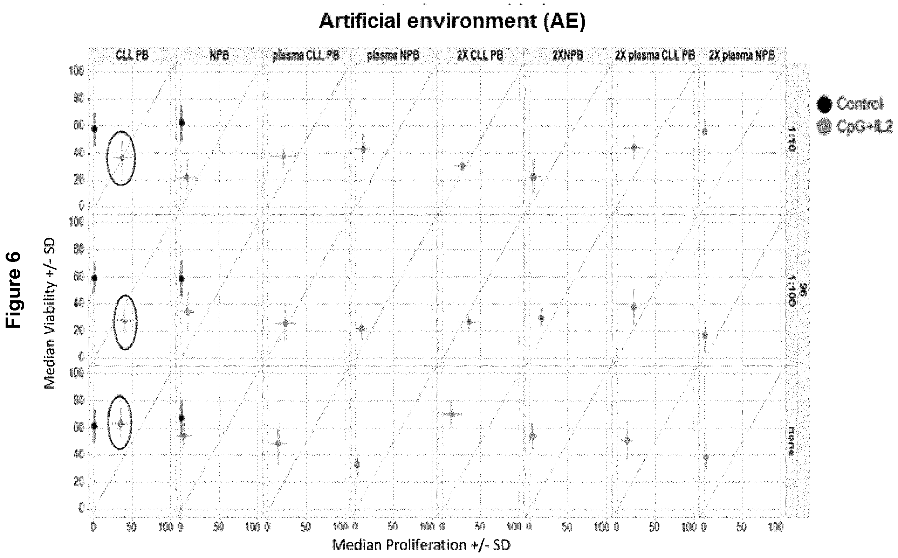

**Figure 6****.** Viability and proliferation using varied AE conditions with, or without cytokines. Using four CLL primary tumor samples, data is compared testing AE from healthy donors (NPB) and from donors with CLL (CLL PB).

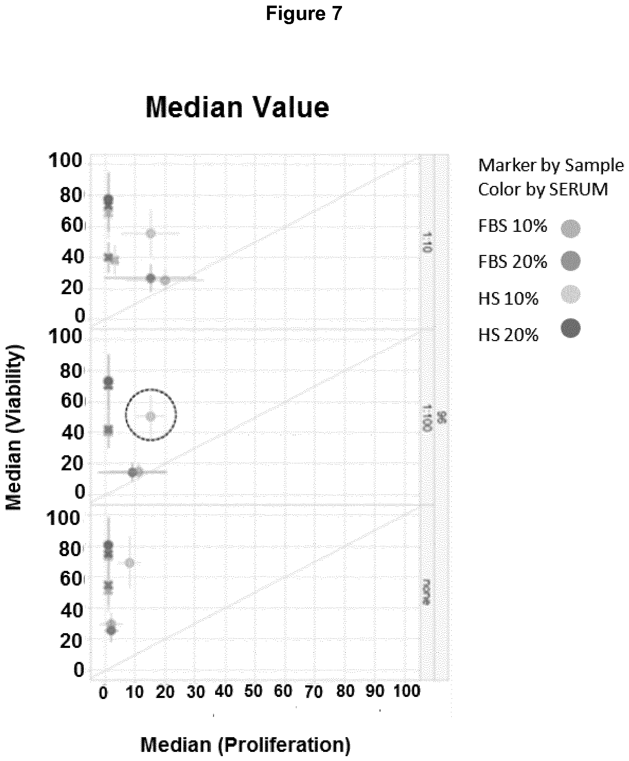

**Figure 7****.** Human Serum (HS) vs. Bovine Serum (FBS). To determine the effect of serum on the assay system, three CLL samples were tested in four different serum conditions using both FBS and HS at two concentrations (10 and 20%). X refers to control and circles correspond to CpG+IL2 combination. The vertical error bars correspond to SD viability and horizontal error bars correspond to SD proliferation. The effect on viability and proliferation is reported.

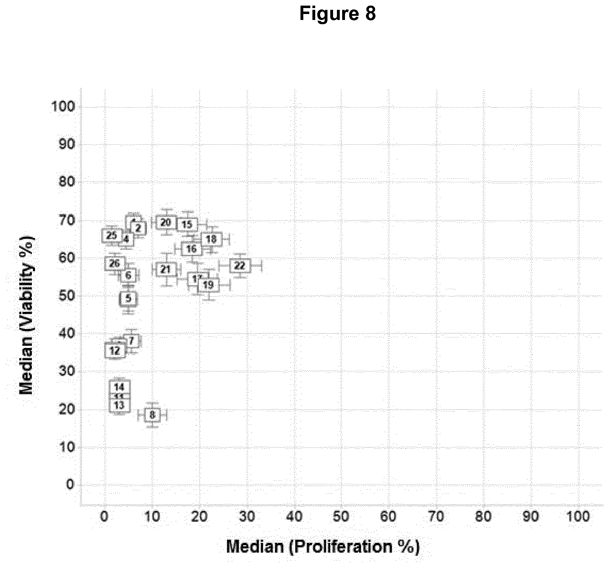

**Figure 8****.** Viability and proliferation using CLLPB +/- HS-5 cells to determine best cytokine protocol. Each cytokine combination tested in 20 CLL samples is represented by the median response for viability and proliferation. The numbers correspond to the cytokine combination indicated in Table 2.

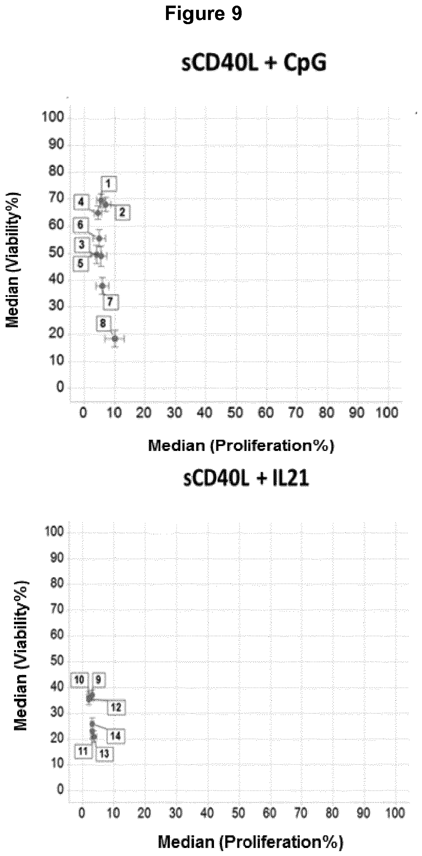

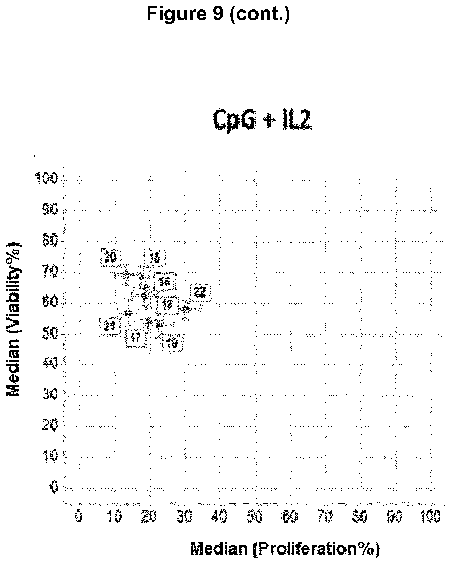

**Figure 9****.** Viability and proliferation of the 24 cytokine combinations of Table 2 grouped by three different backbone stimulations (sCD40L+CpG, sCD40L+IL21, CpG+IL2). The median response for the three different backbone stimulations for each condition is shown.

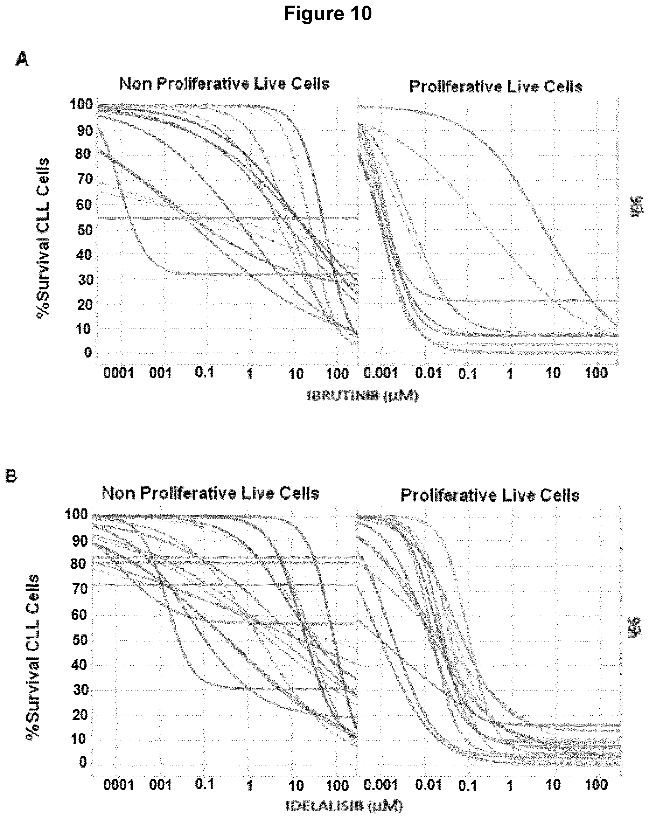

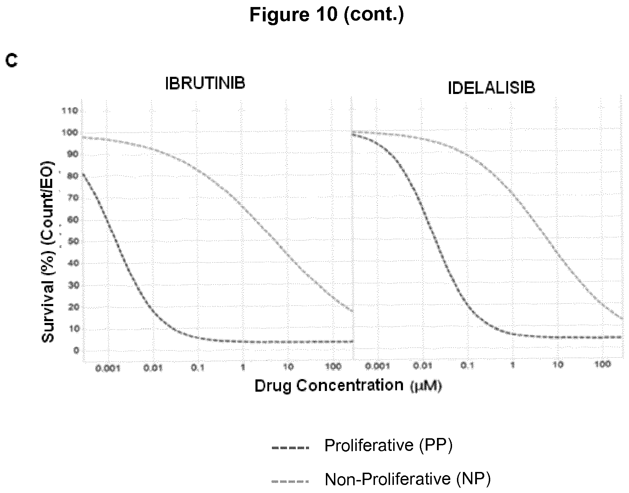

**Figure 10****.** Ibrutinib and Idelalisib antiproliferative activity tested on primary CLL cells in the presence of AE. Dose response curves for Ibrutinib (Fig.10A) and Idelalisib (Fig. 10B) in both the Non-Proliferative (left panel) and proliferative (right panel) cell fraction. Figure 10C represent the median value of both non-proliferative (NP, grey line) and proliferative (PR, dark line) cell subsets for Ibrutinib (left panel) and Idelalisib (right panel).

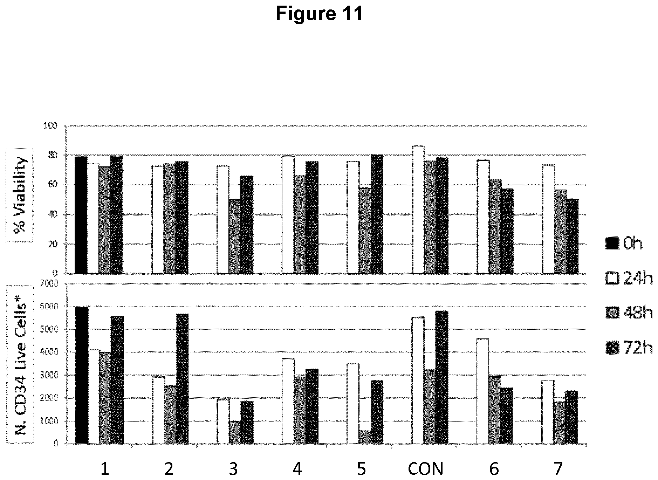

**Figure 11****.** Viability and absolute CD34 blast recovery with different test conditions. Figure 11 represents the percentage of viability (upper panel) or absolute CD34 cell recovery (lower panel) of a MDS sample with 7 different cell culture conditions plus a control, at different time incubations. Numbers refer to conditions specified in Example 10.

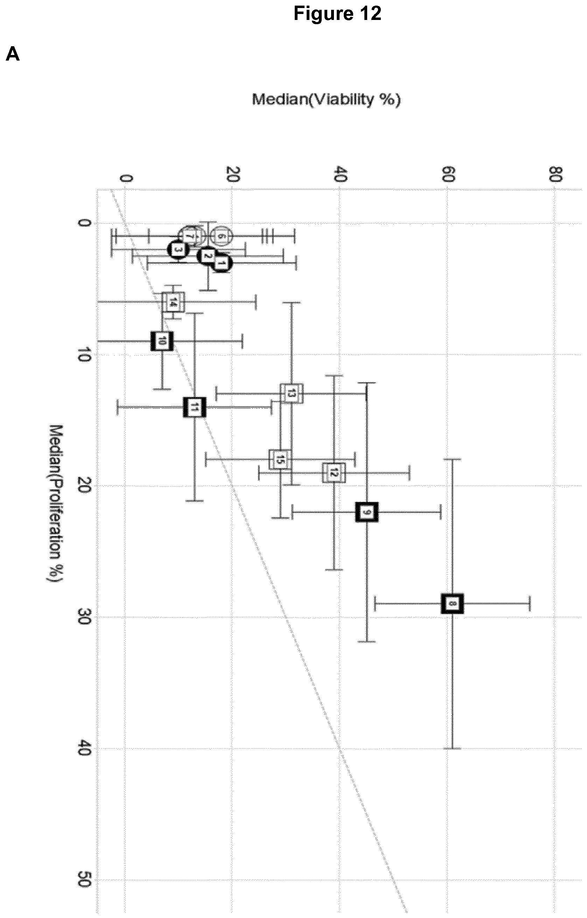

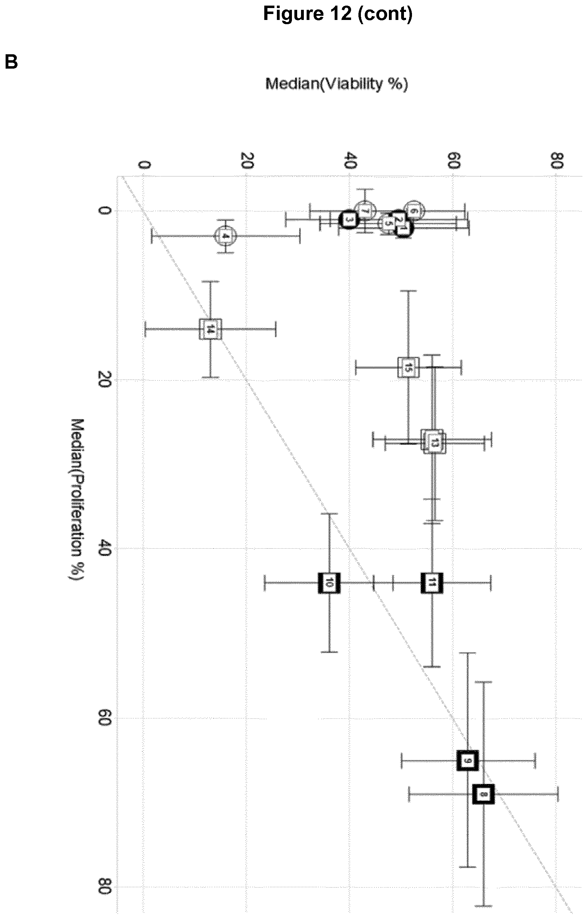

**Figure 12****.** Viability and proliferation in 4 B-NHL primary samples with the cytokine cocktails shown in Table 3, and AE from normal BM. The median response is shown for viability and proliferation in each of the 15 cytokine combinations tested in 4 B-NHL primary tumor samples.
Fig. 12A is the data from a 72h incubation and Fig. 12B is the 96h incubation. Filled squares are combinations containing CD40L, open squares are combinations containing IL2, and circles do not contain cytokines.

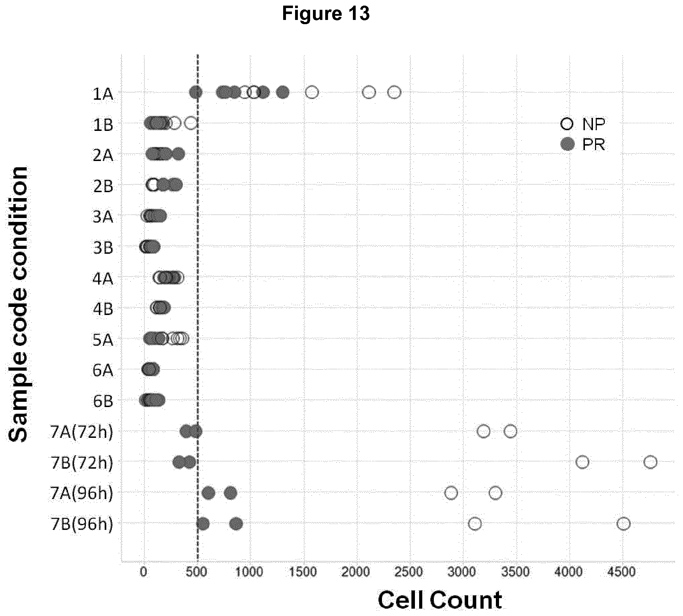

**Figure 13****.** Viability and proliferation in 7 MM primary samples with different test conditions, with or without cytokines. The viability of primary MM samples is shown in both the proliferating (PR, filled circles) and non-proliferating (NP, empty circles) cells under different test conditions summarized in Table 4.

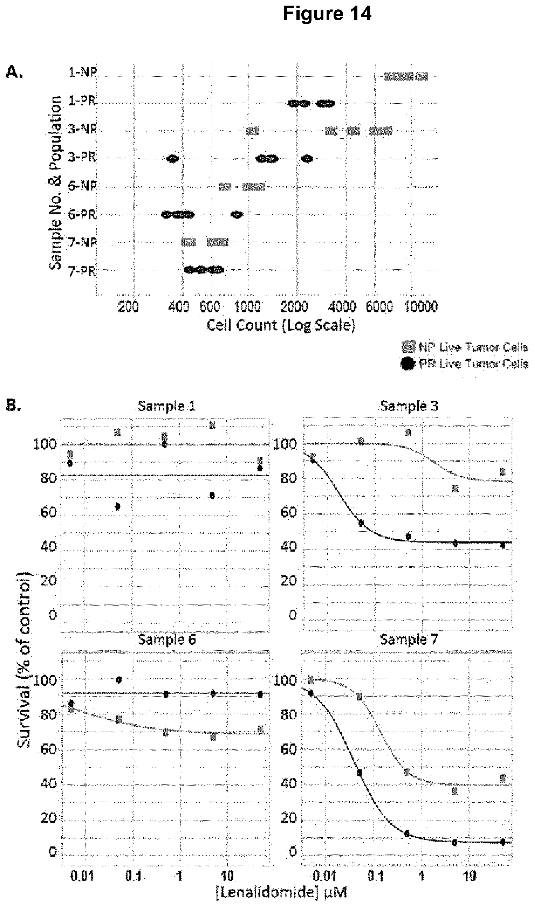

**Figure 14** Lenalidomide anti-proliferative activity tested on primary MM cells in the presence of AE. Figure 14A shows the viability of non-proliferating (NP, grey squares) and proliferating (PR, black circles) cell populations of four representative samples. Figure 14B displays the dose response curves for Lenalidomide in both the non-proliferating (grey line and squares) and proliferating (black line and circles) live MM tumor cell populations from four representative samples.

### Detailed description of the invention

The present invention relates to a method for producing an artificial tumor environment of primary tumor cells and its use in an *ex vivo* method to test the cellular responsiveness of primary tumor cell populations to a drug or combination of drugs. This method is characterized for enhancing and/or promoting the proliferation of a primary tumor cell population in *ex vivo* assays. Isolated primary tumor cells cannot be duly analyzed because they lack certain microenvironmental elements which are critical in promoting the survival and proliferation of a tumor *in vivo.*

We have first defined the method of incorporating the AE for *ex vivo* analysis for the evaluation of responsiveness of primary tumor cells isolated from patients diagnosed with Chronic Lymphocytic Leukemia (CLL), to the presence of anti-tumor drugs in the assay media. Present disclosure, although centered on CLL, represents a technical invention that could be applied to any primary tumor cell population, whether it is from a hematological malignancy or a solid tumor. Included here we also demonstrate the effect of AE on primary tumor cell populations from other hematological malignancies, including Acute Myeloid Leukemia (AML), Acute Lymphoid Leukemia (ALL), Myelodysplastic Syndrome (MDS), non-Hodgkin's Lymphoma (NHL) and Multiple Myeloma (MM)
The present invention also discloses the use of the AE in *ex vivo* assays for determine the effect of a drug (analysis and quantification of the drug effect) on primary tumor cells isolated from patient samples.

Microenvironment - is the environment in which the tumor exists, including surrounding blood vessels, immune cells, fibroblasts, stromal cells, the extracellular matrix (ECM), soluble factors (e.g. tumor derived exosomes, signaling molecules. growth factors, micro RNA, chemokines, cytokines and any soluble molecule derived from tumor or non-tumor cells), all of which affect tumor cell dynamics.

Artificial Environment - (AE, also referred to as AE fraction) Fraction or mixture of fractions isolated from a peripheral blood, or bone marrow, or lymph node sample from a donor after density gradient centrifugation excluding leukocyte fraction (AE leukocyte-free). Residual leukocytes could still remain in the AE. AE can be only the plasma fraction, only the erythrocyte fraction, or a combination of the two fractions at any ratio (e.g. 1:1, 1:2, 2:1, etc.).

AE Leukocyte-free: Fraction or sample without leukocytes, or with a residual number of leukocytes, defined as less than 100 leukocytes per µl of AE.

Donor - Refers to a person who provides a biological sample. The person can be healthy or have been diagnosed with a disease (e.g. cancer). The biological sample can be, for example, blood, bone marrow, lymph node or tumor biopsy.

*Ex vivo -* experimentation or measurements done in or on tissue or cell/s from an organism in an external environment with the minimum alteration of natural conditions. In the present invention refers to primary human cells *in vitro,* from patients suffering a disease or normal individuals, where the cells can be either recently extracted, cryopreserved, or frozen, to preserve their state. In some embodiments, these cells are thawed for the *in vitro* evaluation of drug effects. For the purposes of present patent specification, the term *"Ex vivo"* and *"In vitro"* are used indistinctly.

Hematological Malignancies - (HM) Also called hematological neoplasms, refers to a group of diseases, defined according to the World Health Organization classification, that includes Hodgkin's lymphoma, Non-Hodgkin's lymphoma (e.g., B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), chronic lymphocytic leukemia, acute myeloid leukemia, chronic myeloid leukemia, myelodysplastic syndrome, multiple myeloma, or acute lymphocytic leukemia.

Solid Tumors - consists of neoplasms or lesions formed by abnormal growth of body tissue cells other than blood, bone marrow or lymphatic cells. Solid tumors include, but are not limited to, ovarian cancer, rectal cancer, cancer of the anal region, stomach cancer, testicular cancer, uterine cancer, colon cancer, rectal cancer, renal-cell carcinoma, liver cancer, non-small cell carcinoma of the lung, cancer of the small intestine, cancer of the esophagus, melanoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, carcinoma of the fallopian tubes, brain stem glioma, pituitary adenoma, Kaposi's sarcoma, epidermoid cancer, squamous cell cancer, carcinoma of the endometrium, carcinoma of the cervix, carcinoma of the vagina, carcinoma of the vulva, sarcoma of soft tissue, cancer of the urethra, cancer of the penis, cancer of the bladder, cancer of the kidney or ureter, carcinoma of the renal pelvis, neoplasm of the central nervous system (CNS), primary CNS lymphoma, tumor angiogenesis, spinal axis tumor, metastatic lesions of said cancers, or combinations thereof.

Sample - Also called patient sample, primary tumor cells, isolated tumor cells, primary cells, all refer to primary cells that have been extracted from a patient and have been isolated for analysis. A sample can be primary cells from a healthy individual, a patient diagnosed with cancer, or a patient diagnosed with another hematological disorder. A sample may or may not have been cryopreserved. Cryopreserved samples have to be thawed before use.

ExviTech - Automated and proprietary flow cytometry platform that enable pharmacological characterization of drugs and drug combination treatments in the context of microenvironmental stimuli, directly on patient samples to assess response (15).

Primary tumor cells - Refers to tumor cells taken directly from living tissue (e.g. bone marrow, peripheral blood, lymph nodes, spleen, or tumor biopsy), isolated and established for *ex vivo* growth. Primary tumor cells may have been previously extracted and cryopreserved and thawed before use, or may be recently extracted and used without cryopreservation.

Primary cell population - Refers to cells (non-diseased) taken directly from living tissue (e.g. bone marrow, peripheral blood, lymph nodes, spleen, or tumor biopsy) that are established for *ex vivo* growth.

Stromal cell population representative of a cell type found in the *in vivo* tumor microenvironment. The tumor microenvironment is composed of several non-tumor cell types that can include, but is not limited to, fibroblastic reticular cells, nurse-like cells, mesenchymal stem cells, follicular dendritic cells, endothelial cells and pericytes. The stromal cells can be primary cells, or cells derived from primary stromal cells including, but not limited to, HS-5, HS27a, UE6E7T-2, NK-Tert, HMEC-1, KUSA-H1, M210B4, ST-2, and KUM-4.

Density Gradient Centrifugation - Refers to a process that separates a blood, or bone marrow, or sample into fractions of discrete components, e.g. plasma, erythrocytes, granulocytes and monocytes. Blood, or bone marrow, or lymph node samples are layered over a gradient medium (e.g. Ficoll-Paque™, Lymphoprep™, Histopaque-1077™, or other similar mediums) and after centrifugation the isolated fractions of each component can be removed (Fig. 1).

Plasma fraction or Serum fraction - Plasma obtained from a blood, or bone marrow, or lymph node sample after density gradient centrifugation. This is the top most fraction, as seen in Figure 1. Residual leukocytes could still remain in this fraction, but at a concentration of less than 100 leukocytes/ µl AE. Serum is the plasma, with the clotting factors removed.

Erythrocyte fraction - AE comprising mainly erythrocytes. When a sample of peripheral blood, bone marrow or lymph node is separated into various component parts by density gradient centrifugation, this is the bottom fraction as see in Fig. 1. Residual leukocytes could still remain in this fraction, but at a concentration of less than 100 leukocytes/ µl AE.

Soluble factors - Refers non-cellular components of the tumor microenvironment that can include tumor derived exosomes, signaling molecules, growth factors, chemokines, cytokines, micro RNA, and any soluble molecule derived from tumor or non-tumor cells (*9*, *16, 17*). Examples of these can include, but are not limited to, Interleukin (IL)-2, IL-4, IL-6, IL-8, IL-10, IL-13, IL-15, IL-21, soluble cluster of differentiation (sCD) 40 ligand, Immunoglobulin (Ig) M, CpG (short synthetic single-stranded DNA molecules containing non-methylated CpG dinucleotides motifs), B cell activating factor (BAFF), a Proliferation Inducing Ligand (APRIL), Tumor derived exosomes (tumor cell-derived vesicles that are present in the tumor microenvironment).

Types of Drugs - chemotherapeutic or cytotoxic drug, medicines that contain chemicals which are toxic to cells, preventing their replication or growth (e.g. busulfan, cyclophosphamide, mitoxantrone, daunorubicin, doxorubicin, melphalan, vincristine, vinblastine and chlorambucil); targeted anti-cancer therapy drug, drugs designed to interfere with specific molecules necessary for tumor growth and progression (e.g. imatinib, dasatinib, nilotinib); oncolytic drug, a virus that invades tumors and replicates itself, thereby killing cancer and spurring an immune response to multiply its effect (e.g. Imlygic and Peptichemio); immunotherapy drug, treats disease by inducing, enhancing, or suppressing an immune response (e.g. Alentuzumab, Ofatumumab, Rituximab); cytokine therapy, a broad category of small proteins that are important in cell signalling that can be used to treat disease (e.g. interferons (IFNα, IFNβ, IFNγ and IFNλ) and interleukins (IL-2, IL-8, IL-21); tumor microenvironment drugs, drugs that target the microenvironment rather than the tumor (e.g. Ibrutinib and idelalisib); activator of a costimulatory molecule, agents that activate co-stimulatory molecules that are crucial to the development of an effective immune response (e.g. Cluster of differentiation (CD)28, OX40, CD137, CD27, Herpesvirus-entry mediator (HVEM)); inhibitor of an inhibitory molecule, drugs that evokes a response by silencing inhibitors of a system (e.g. antibodies to programmed cell death 1 (PD1) and Lymphocyte-activation gene 3 (LAG-3)).

Parameters that may be measured - viability, measurement of live cells; proliferation, the formation or development of cells; apoptosis, the process of programmed cell death; cell depletion, loss of cells through mechanisms that are not necrosis or apoptosis; changes in surface marker expression, monitoring the appearance or disappearance of cellular surface markers such as the CD markers; changes in cytokine/chemokine production, measuring levels of these molecules that are produced by a cell population.

3D Cell Culture - an artificially-created environment in which biological cells are permitted to grow or interact with their surroundings in all three dimensions (e.g. spheroid constructs, extracellular matrix gels, synthetic scaffolds, rotary cell culture systems, or on low/non-adherent culture plastics (*15, 18, 19*).

Circulating Tumor Cells - cells that break away from a tumor and move through a cancer patient's bloodstream.

Whole sample (e.g. whole peripheral blood, whole bone marrow or whole lymph node) - The use of the sample in its entirety. No components have been removed or isolated from the sample. As an example, lymphocytes isolated from a bone marrow sample are not considered whole sample.

The use of AE to test of the responsiveness of primary tumor cells populations to a drug or combination of drugs method disclosed in the present invention has several advantages over that of the prior art that are described herein. One advantage is that the method of present invention enhances the viability of a primary tumor cell population, thus prolonging the duration of the tests and the corresponding assessment of drug long term activity. Another advantage is that the method promotes the proliferation of a primary tumor cell population, to levels that have not been previously reported (14). These advantages allow the use of a large number of variables in an *ex vivo* analysis of the cellular response of the primary tumor cell population, including different incubation times and many drug compositions such as drugs that inhibit cell proliferation. Another advantage is the capacity to analyze the primary tumor cell population in a manner that more closely mimics the *in vivo* microenvironment of the primary tumor cell population in a patient. This is particularly important in the case of drugs targeting the microenvironment e.g. Ibrutinib and Idelalisib, whose assessment requires microenvironment-mimicking assays. Combined with previously mentioned features and advantages of the method, the present invention also affords the advantage of improving the ability of an *ex vivo* test to determine if a drug or drugs would be beneficial to a patient (or patients) in the treatment of primary tumors. In a particular embodiment, this test can be used determine the effectiveness of a given treatment in a patient.

In an embodiment, AE (e.g. plasma fraction only, erythrocyte fraction only or both, but always excluding leukocyte fraction, AE leukocyte-free) is added to *ex vivo* assays that test the cellular responsiveness of primary cells to a drug or drugs. In another embodiment, AE (e.g. plasma fraction only, erythrocyte fraction only or both, but always excluding leukocyte fraction, AE leukocyte-free) is added to *ex vivo* assays that test the cellular responsiveness of primary tumor cells to a drug or drugs. In an embodiment, the AE contains a residual number of leukocytes less than 100 per µl of AE. In another embodiment, the residual leukocytes present in an AE preparation are labeled to distinguish them from primary tumor cells to be tested. In one embodiment, the primary tumor cells are incubated in an incubation mixture comprising primary tumor cells, AE and a drug or drugs. In another embodiment, the primary tumor cells are incubated in an incubation mixture comprising AE, one or more soluble factors (e.g. cytokines/chemokines or molecules derived from non-tumor cells) and a drug or drugs. In another embodiment, the primary tumor cells are incubated in an incubation mixture comprising AE, a co-culture of an additional cell population that is representative of a cell type found in the *in vivo* tumor microenvironment, and a drug or drugs. In a further embodiment, the primary tumor cells are incubated in an incubation mixture comprising AE, one or more soluble factors (e.g. cytokines/chemokines or molecules derived from non-tumor cells), and a co-culture of an additional cell population that is representative of a cell type found in the *in vivo* tumor microenvironment, and a drug or drugs.

In an embodiment, AE is added to *ex vivo* assay systems that contain components from other species. These components include, but are not limited to, fetal bovine serum, calf bovine serum, or components derived from these. In one embodiment, AE is added to *ex vivo* assays that contain culture media supplemented with different nutrients. These media include, but are not limited to RPMI-1640, AIM-V, DMEM and IMDM. In a further embodiment, AE is added to assay systems comprised of *ex vivo* 3D cell culture constructs built to mimic the microenvironment architecture of solid tumors. This is achieved for example by culturing primary tissues or established cell lines within spheroids, extracellular matrix gels, synthetic scaffolds, rotary cell culture systems, or on low/non-adherent culture plastics (*18-20*).

In an embodiment, the incubation mixtures are incubated for a period of 2 hours to 14 days prior to the analysis of the effect of the drug or drugs on the primary tumor cells. In an embodiment, the soluble factors added to the incubation mixture are comprised from a list including, but not limited to tumor derived exosomes, signaling molecules, growth factors, chemokines, cytokines and any soluble molecule derived from tumor or non-tumor cells (*9*, *16,* 17). Examples of these can include, but are not limited to, IL-2, IL-4, IL-6, IL-8, IL-10, IL-13, IL-15, IL-21, sCD40L, IgM, CpG, BAFF, or APRIL.

In an embodiment, the additional cell population that is representative of a cell type found in the *in vivo* tumor microenvironment added to the incubation mixture is comprised from a list including, but not limited to either primary cells or cell lines derived from: fibroblastic reticular cells, nurse-like cells, mesenchymal stem cells, follicular dendritic cells, endothelial cells, and pericytes (*5*, *21, 22*)*.* Cell lines derived from primary stromal cells can include, but are not limited to, HS-5, HS27a, UE6E7T-2, NK-Tert, HMEC-1, KUSA-H1, M210B4, ST-2, and KUM-4.

In a preferred embodiment, the AE fractions are collected from several donors, all different from the patient providing the primary tumor cells, wherein all plasma fractions have been pooled together and erythrocytes from compatible blood types have been pooled, reducing the interpatient donor variability in the AE. In another embodiment, the AE fractions are comprised of fractions from a single donor, different from the patient providing the primary tumor cells. In a further embodiment, the AE fractions are comprised of fractions obtained from the same patient that provides the primary tumor cells.

In a preferred embodiment, the AE is comprised of fractions obtained from donors/patients with the same type of cancer as the patient from which the primary tumor cells to be tested have been extracted (e.g. to test a primary tumor sample from a patient diagnosed with CLL, the AE used in the assays would be comprised of plasma fractions and serum fractions obtained from one or more patients also diagnosed with CLL). In another embodiment, the AE is comprised of fractions obtained from healthy donors. In a further embodiment, the AE is comprised of fractions obtained from donors diagnosed with a cancer different from the cancer of the primary tumor cells to be tested.

In an embodiment, the AE corresponding to a primary cell sample is preserved separately, whereby plasma fraction can be frozen at -80 °C and the erythrocyte fraction is preserved in CPDA bags for up to 45 days at 4°C.

In a preferred embodiment, the primary cell sample has been cryopreserved prior the test. In another embodiment, the primary cell sample has been freshly extracted. In a particular embodiment, the cryopreserved primary cell sample is thawed together with the AE. In a preferred embodiment, the cryopreserved primary cell sample is cryopreserved together with the AE. Primary cell samples can have been cryopreserved for any length of time after freezing. In one embodiment, a primary cell sample is cryopreserved from 5 days to 1 year after freezing. In another embodiment, a primary cell sample is cryopreserved from 1 year to 20 years after freezing. The reconstitution of the cryopreserved primary cell sample with the AE provides a sample more similar to the native environment of said primary cells and increase viability and/or proliferation and/or maintaining of the pharmacological properties of the primary cells.

In one embodiment, the primary tumor sample has been extracted from an adult cancer patient. In another embodiment, the primary tumor sample has been extracted from a pediatric patient. In an embodiment, the primary tumor sample has been extracted from a cancer patient that has not received a prior treatment for the cancer. In another embodiment, the primary tumor sample has been extracted from a cancer patient that has received one or more previous treatments for the cancer. In a further embodiment, the primary tumor sample has been extracted from a patient with Minimal Residual Disease (MRD).

In a specific embodiment, the primary tumor cells are obtained from a patient diagnosed with a hematological malignancy. Hematological malignancies include, but are not limited to, Hodgkin's lymphoma, Non-Hodgkin's lymphoma (e.g., B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), chronic lymphocytic leukemia, acute myeloid leukemia, chronic myeloid leukemia, myelodysplastic syndrome, multiple myeloma, or acute lymphocytic leukemia. In a preferred embodiment, the primary tumor cells from a patient diagnosed with a hematological malignancy is selected from a group consisting of peripheral blood, bone marrow, lymph node, and spleen.

In another embodiment, the primary tumor cells are extracted from a patient diagnosed with a solid tumor. Solid tumors include, but are not limited to, ovarian cancer, rectal cancer, cancer of the anal region, stomach cancer, testicular cancer, uterine cancer, colon cancer, rectal cancer, renal-cell carcinoma, liver cancer, non-small cell carcinoma of the lung, cancer of the small intestine, cancer of the esophagus, melanoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, carcinoma of the fallopian tubes, brain stem glioma, pituitary adenoma, Kaposi's sarcoma, epidermoid cancer, squamous cell cancer, carcinoma of the endometrium, carcinoma of the cervix, carcinoma of the vagina, carcinoma of the vulva, sarcoma of soft tissue, cancer of the urethra, cancer of the penis, cancer of the bladder, cancer of the kidney or ureter, carcinoma of the renal pelvis, neoplasm of the central nervous system (CNS), primary CNS lymphoma, tumor angiogenesis, spinal axis tumor, metastatic lesions of said cancers, or combinations thereof. In a preferred embodiment, the primary tumor cells from a solid tumor are obtained via a biopsy of the solid tumor. In another embodiment, the solid tumor cells are circulating tumor cells extracted via a peripheral blood sample. In an embodiment, the drug or drugs tested in an incubation mixture containing AE, consist of one or more therapeutic agent or agents chosen from, but not limited to, the following categories: chemotherapy, a targeted anti-cancer therapy, an oncolytic drug, a cytotoxic agent, an immune-based therapy, a cytokine, drugs targeting the microenvironment, an activator of a costimulatory molecule, an inhibitor of an inhibitory molecule, or a cellular immunotherapy.

In one embodiment, the *ex vivo* analysis of the cellular response of the primary tumor cell population includes, but is not limited to, measuring one or more of the following: viability, proliferation, apoptosis, cell depletion, changes in surface marker expression, changes in cytokine/chemokine production, and analysis of these parameters on one or more subpopulations of cells identified within the primary tumor cell population.

In an embodiment, AE (e.g. plasma fraction only, erythrocyte fraction only or both) is added to *ex vivo* assays that test the cellular responsiveness of primary cells, cells that are not tumor cells, to a drug or drugs. The primary cells may be extracted from patients diagnosed with other hematological disorders, including but not limited to, autoimmune diseases, blood coagulation disorders, and anemia. In a particular embodiment, primary tumor cells are extracted from a patient after treatment of the disorder has been started. The *ex vivo* assay that test the cellular responsiveness of primary tumor cells can be used to determine if a prescribed treatment is effective to said patient. In an embodiment, the patient sample is obtained 5, 6, 7, 8, 9, or 10 days after initiation of treatment. In a particular embodiment, the sample from the patient corresponds to a lymph node biopsy obtained after being treated with idelalisib 7 days after the start of treatment.

In an embodiment, AE (e.g. plasma fraction only, erythrocyte fraction only or both) is added to *ex vivo* assays that test the cellular responsiveness of primary cells, either healthy or pathological, to a drug or drugs that are in development.

In an embodiment, AE (e.g. plasma fraction only, erythrocyte fraction only or both) is added to *ex vivo* assays that test the cellular responsiveness of primary cells to a drug or drugs being tested in a clinical trial. In this embodiment, results could provide information on the whether the drug is potentially beneficial to a patient, and if the patient would be a good candidate for the clinical trial.

### EXAMPLES

The PB, BM and LN samples used in this document were from adult patients over 18 years of age. All patients gave informed written consent for study participation using protocols approved by the ethical committees of each hospital. Samples were provided by Spanish Centers from the Programa Español de Tratamientos en Hematologia (PETHEMA) group and Hospital San Raffaelle, Milan, Italy.

### Example 1 - Preparation of Artificial Environment (AE) components by density gradient centrifugation

The components of the AE described in this invention are prepared as follows: Histopaque 1077 (Sigma) is added to a conical centrifuge tube. The peripheral blood (PB), or bone marrow (BM), or lymph nodes (LN) samples from donors are carefully layered onto the Histopaque-1077 solution and centrifuge at 1500 rpm for 40 minutes. The upper layer, containing the plasma fraction is removed first. The middle layers containing the mononuclear cells (MNC), Histopaque-1077 and granulocytes layers (leucocyte fraction) are removed and discarded. The bottom layer containing the red blood cells (RBC) or erythrocyte fraction is then removed (Figure 1). The plasma fractions are stored at -80°C until use. The anticoagulant citrate phosphate dextrose adenine solution (CPDA-1, Terumo Corporation, Tokyo, Japan) is added to the RBC fraction (150µl CPDA/ml RBCs), and this is stored at 4°C for a maximum of 35 days. The plasma and erythrocyte fraction are mixed in a 1:1 proportion for all experiments where AE is supplementing the cell media.

The middle sections are excluded to remove the gradient media and the leukocyte population as well. The exclusion of the leukocytes allows for the use of an AE preparation in tests of primary cell samples from other donors without the worry of Major Histocompatability Complex (MHC) interactions. The MHC receptors on some of the leukocyte populations (e.g. T cells and NK cells) may cause them to attack primary cells from non-autologous samples. Thus it is necessary to exclude the leukocyte population from the AE preparation. However, the method described is in practice an enrichment process. All fractions may contain a residual number of leukocytes, but not enough to have a measurable MHC effect on the primary cells populations being tested. Using an AE composed of plasma and erythrocytes adds factors to an *ex vivo* assay system that can impact the viability of primary cells and their ability to proliferate. These AE factors also may affect how a drug interacts with the primary cells in terms of efficacy and potency.

In case of using only the plasma fraction, the AE is prepared as follows: The whole PB, BM or LN sample is centrifuged at 2500 rpm for 15 minutes at 4°C. The upper layer containing the plasma fraction is removed, aliquoted in small fractions and stored at -80°C until use.

### Example 2 - AE Enables Good Correlation of Results Between Fresh and Frozen Primary Tumor Cells

The role of AE in thawing previously cryopreserved samples was investigated. Cryopreservation methods commonly rely on a density gradient to isolate the leukocytes, and only the leukocyte fraction is cryopreserved. As a consequence, when these cells are thawed the result is isolated leukocytes devoid of its AE. The activity of compounds on these thawed cells could thus be artificially modified by the absence of the AE. We have investigated whether we can add back the AE of these cryopreserved cells, and whether the freeze/thaw process alters the compound activity of both cytotoxic and antiproliferative drugs. Fresh BM AML samples were divided into two parts; in one part, we evaluated the dose response curves of several compounds in its AE as fresh samples. In the other part, we performed a density gradient isolating the leukocyte fraction that was cryopreserved, the plasma fraction that was frozen at -20°C, and the erythrocyte-granulocyte fraction that was preserved in CPDA from blood bags at 4°C. After 2 weeks, the cryopreserved vials were thawed, and the plasma and erythrocyte fractions from each patient were mixed in a 1:1 ratio and added to the thawed primary cells. We repeated the same evaluation of the activity of these compounds and compared the results in fresh vs frozen samples of the same patients with reconstituted AE.

Eight concentrations of seven different drugs were tested and incubated for 24 hours and 48 hours, and the results of the viability prior to incubation were also obtained. After incubation, red blood cells were lysed and leukocytes stained using a combination of monoclonal antibodies to identify leukemic cells. Annexin V was also included to exclude apoptotic cells from the analysis. Area under the curve (AUC) was calculated for each drug and each condition. Figure 2A shows the correlation between AUC obtained for each drug (different color intensity) both at 24 h and 48 h (filled vs empty circles), using fresh and frozen primary AML cells. A good correlation was obtained for each drug and condition (r²= 0.618). This indicates that the activity of these compounds was not affected by freezing and thawing these cells, as long as the AE was restored. Thus, cryopreserved samples with reconstituted AE can also be used to evaluate compound activity.

Figure 2B shows the differences in the viability obtained when the same AML sample is processed with different assays. Comparison of the viability of leukemic cells in fresh whole bone marrow with frozen and then thawed isolated leukocytes from the same sample, was assayed in 5 AML samples (grey boxes). Comparison of the viability of leukemic cells in fresh whole bone marrow with frozen and then thawed isolated leukocytes from the same sample with reconstituted AE, was assayed in 10 AML samples (black boxes). Upper panel represent the initial viability of leukemic cells whereas lower panels show the viability of leukemic cells after 48 h incubation. Median, average (Avg) and standard deviation (StdDev) of each condition is showed in a table below each graph. No significant differences are observed in the initial viability for each condition. However, a decrease of viability is observed when isolated leukocytes without reconstituted AE are used, compared to whole bone marrow. This decrease is not observed once AE is added to the isolated leukocytes, getting a very good viability, similar to that obtained in the fresh whole bone marrow.

Similar results were observed with other hematological malignancies such as ALL. Figure 2C shows the Dose Response curves at 48h and 72h for a compound in an ALL sample. Solid line represents the fresh sample and the dotted line represents the thawed isolated lymphocytes from the same sample reconstituted with cryopreserved AE from the patient (plasma and erythrocyte fraction). As seen in Figure 2C, no significant differences were observed between both methods indicating that AE could be added later to thawed samples obtaining similar drug sensibility.

### Example 3 - Assay to test Viability and Proliferation in Primary Tumor Cell Population

For all assays testing primary tumor cell populations, the Vybrant® CFDA SE Cell Tracer Kit (Molecular Probes) is used to measure cell proliferation. The CFDA SE (component A) is dissolved in dimethylsufoxide (component B) at a concentration of 5 mM as stock solution and kept at -20°C until further use. In this example, freshly extracted and isolated CLL cells are adjusted to 10x10⁶ cells/ml in phosphate buffer saline (PBS, Sigma). CFDA is added to a 1 ml cell suspension of primary CLL cells, to a final concentration of 5 µM. After addition of CFDA, cells are vortexed and incubated at room temperature for 10 min with continuous shaking and light protected. At the end of the incubation period, the cells are resuspended in media and kept on ice for 5 min. The cells are washed twice with media and maintained at 4°C until use. This step is performed prior all other steps in the experimental preparation.

The CFDA labeled primary CLL cells are then dispensed into 96-well plates. Cells are stimulated with the cytokine combination of 1µg/ml Oligodeoxy-ribonucleotides containing CpG motifs (ODN2006; Miltenyi Biotec, Bergisch Gladbach, Germany) plus 50 ng/ml Interleukin-2 (IL-2, Peprotech, London, United Kingdon). No stimulation is also used as a control of both viability and proliferation. After 96 h of incubation the plates are washed. Then 20 µl of a combination of Annexin-V CF Blue (Immunostep, Salamanca, Spain), CD19 PC7 (Clone J4.119, Beckman Coulter) and CD45-APC (clon HI30, Immunostep, Salamanca, Spain) is added. After 15 minutes of incubation at room temperature in the dark, a wash step is performed and the pellet is resuspended in 80 µl of buffer for analysis in Vivia's ExviTech platform.

For analysis, the antibodies CD19 and CD45 are used to identify the primary CLL cells, Annexin-V staining discerns between apoptotic and live cells to test viability, and CFDA allows for proliferation to be monitored. Figure 3A shows how primary CLL cells are identified using a gating strategy based on forward scatter (FSC) and/or side scatter (SSC) and the expression of different surface markers (CD19 and CD45). Figure 3B shows a plot displaying the primary CLL cells identified in A, but where Annexin-V staining discerns between apoptotic and viable cells. Figure 3C displays how CFDA staining identifies the proliferating population. The left panel shows an example of CLL cells that have not been exposed to cytokines, and still shows a single population. The right panel displays CLL cells exposed to cytokines in which part of the sample is proliferating and part of the sample is not.

### Example 4 - Viability and Proliferation in CLL Samples Supplemented with AE from Normal PB or BM, Co-cultured with HS-5 Stromal Cells

A flow cytometry based screening process is used to test for the optimal conditions for primary Tumor cells from patient diagnosed with CLL. The conditions tested are summarized in Table 1. The ratio of HS-5 stromal cells to B-cells in each assay is indicated.

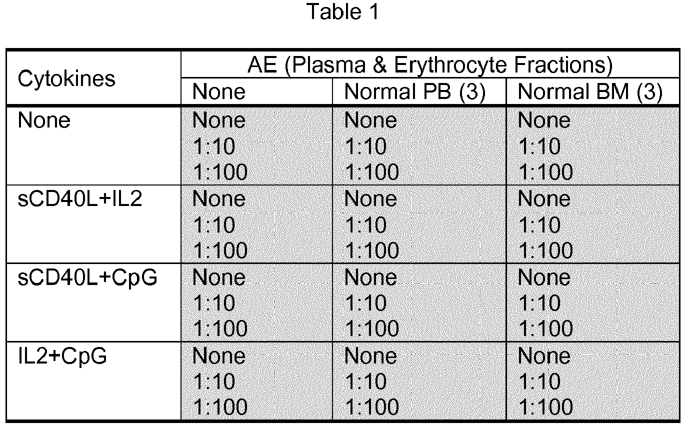

Three cryopreserved CLL primary tumor samples are tested with and without the presence of HS-5 stromal cells (none, 1:10 and 1:100). Plates that incorporated HS-5 (23) cells are plated at either 10² or 10³ cells/60 µl/well in 96-well plates and incubated for 24 hours to allow cells to adhere. The CLL primary tumor cells are labeled with CFDA. Any residual leukocytes that are present in the AE will not be labeled with CFDA and in this manner can be differentiated from the primary tumor cell population being tested.

The AE, the plasma and erythrocyte fractions, are isolated from 6 healthy donors, three from PB and three from BM, and are tested separately (samples were not pooled). The AE is added to the appropriate wells at 1 µl/well. Three cytokine pairs are also tested: 1 µg/ml CD40 Ligand (sCD40L, Peprotech, London, United Kingdon) plus 25 ng/ml IL-2 (Peprotech, London, United Kingdon), 1 µg/ml sCD40L plus 1 µg/ml CpG (ODN2006 Miltenyi Biotec, Bergisch Gladbach, Germany) and 1 µg/ml CpG plus 50 ng/ml IL-2. No stimulation is also used as a control of both viability and proliferation. Replicate plates for each condition are incubated for 2 time points, 72 hours and 96 hours, each in triplicate.

The experiment tested both viability and proliferation of the malignant B-cell population in the CLL patient samples. The overall results are shown in Figure 4, where each graph shows viability on the Y-axis and proliferation on the X-axis measured as the median viability +/- SD (Standard deviation). For each time point, 72 h or 96 h, the vertical columns display the absence of co-culture with HS-5 cells (0), co-culture with a 1:10 (1:10) or a 1:100 (1:100) ratio of HS-5 cells to tumor cells. The three horizontal rows show AE conditions, the top row does not contain AE, the middle row contains AE from normal BM and the bottom row is AE from normal PB. Figure 4 shows the median response for each cytokine combination. The highest levels of proliferation are samples that do not contain the AE (top row). This shows that testing the effect of a drug on isolated cells without the AE components may overestimate the effectiveness of a drug, as the AE may have components that also affect the tumor cells. If a drug primarily affect is on proliferating cells, it would have a larger ex vivo effect in the absence of AE. While many conditions improved viability, it is more difficult to induce proliferation. Of all the conditions tested, only the CpG + IL-2 cytokine pair had a positive effect on both viability and proliferation (See circles). This is seen at 96 hours in the presence of 1:100 co-culture with HS-5 cells, either without AE, or with AE derived from NPB. It appears that AE derived from NBM inhibits the proliferation induced by CpG+IL-2. The best overall condition considering both viability and proliferation was a 96h incubation, with CpG + IL-2 and AE from NPB co-cultured with HS-5 cells at 1:100 (Fig. 4 bottom right).

### Example 5 -Viability and Proliferation Using AE from PB from Healthy Donors and from CLL Patient Samples

The present methods were used to evaluate AE derived from the PB of both healthy donors and CLL patients. Conditions for this experiment were set using 1 µg/ml CpG plus 50 ng/ml IL-2 to stimulate the samples both without HS-5 co-culture and with a 1:10 HS-5 co-culture, each at 72-hour and 96-hour incubation times. Cryopreserved primary CLL tumor cells from a representative patient sample is shown in Figure 5. Open bars are the 72 h results and the closed bars are the 96 h results. Control samples refer to the CLL primary tumor sample without AE, plus or minus CpG+IL2. The primary tumor sample was tested with AE (plasma and erythrocytes fraction, 1 µl/well) from 5 healthy donors (NPB) and from 5 CLL donors (CLL). As can been seen in the top set of graphs, viability levels were fairly close between the 2 types of AE, with the presence of the HS-5 co-culture somewhat lowering the levels for both. The lower graphs show levels of proliferation, where the biggest impact is with HS-5 co-culture and AE from CLL patients (lower right for). These conditions provided levels of tumor cell proliferation up to 60%. This is well above proliferation levels of only 15% in primary CLL cells that have been recently reported (14). One other point of interest is that within similar donor samples, the results still vary. This is significant in one particular AE that greatly inhibited the viability of the CLL tumor cells with HS-5 co-culture (see circle). This indicates that the AE obtained will need to be tested, and potentially pooled before use to limit the inter-patient variability seen here.

### Example 6 - Viability and Proliferation using pooled AE with plasma only or with plasma + erythrocyte

The present methods were used to evaluate the effect of the different component of the AE from CLL or normal samples in CLL cryopreserved samples. Four CLL frozen samples were seeded with 1 µg/ml CpG plus 50 ng/ml IL-2 (grey circles) together with controls (black circles) and both proliferation (X-axis, Median proliferation +/- SD) and viability (Y-axis, median viability +/- SD) were tested (Figure 6). Vertical and horizontal lines from the circles represent the error bar for both parameters. Six Normal PB (NPB) and six CLL PB media were used as source of AE supplement. To avoid high interpatient variability, the plasma and RBC for all the 6 samples adding all together in the same reservoir keeping the proportion (1:1) as previously described. The three horizontal rows show different HS5 stromal cell line density (1:10, 1:100 and none) at 96-hour incubation. Each column represents different cell culture conditions:
- First column (CLL PB): AE composed of plasma and erythrocytes (RBC) obtained from Peripheral blood from CLL).
- Second column (NPB): AE composed of plasma and erythrocytes obtained from Normal peripheral blood.
- Third column (plasma CLL PB): only the plasma fraction (without RBC) of CLL PB s.
- Fourth column (plasma NPB): only the plasma fraction (without RBC) of the NPB samples.
- Fifth column (2x CLL PB): double of amount of the AE composed of plasma and erythrocytes obtained from CLL PB.
- Sixth column (2x NPB): double of amount of the AE composed of plasma and erythrocytes obtained from NPB.
- Seventh column (2x plasma CLL NPB): double amount of the plasma fraction of the CLL samples.
- Eighth column (2x plasma NPB): double amount of the plasma fraction of the NPB samples.

These conditions show a clear difference between adding AE composed of plasma and erythrocytes from CLL PB (column 1) vs NPB (column 2). The AE from the CLL PB plus cytokines increased proliferation (see circles) while the AE from normal patients (NPB) had little effect on proliferation, even in the presence of cytokines. In addition, for the CLL derived AE it seems to be relevant to add AE composed of plasma and erythrocytes (columns 1 and 5) rather than only the plasma fraction (columns 3 and 7), as when only the plasma was used both proliferation (X-axis) and viability (Y-axis) are diminished. There appears to be a saturating effect with the amount of AE that is added as well. Adding higher concentrations as in columns 5-8 showed no marked improvement over what is seen in columns 1-4. These experiments demonstrate the importance of AE to improve proliferation and viability, especially with AE derived from CLL PB, where some of the components seem to increase these parameters in comparison with NPB.

### Example 7 - Human Serum vs. Fetal Bovine Serum

The media that the primary CLL tumor cells are maintained in during the incubation process contains 10% Fetal Bovine Serum (FBS). To determine the effect of FBS on the assay system, three CLL samples were tested in four different serum conditions for the incubation media, 10% FBS, 20% FBS, 10% Human Serum (HS), or 20% HS. The CLL tumor samples were tested either without HS-5 co-culture, or with co-culture at ratios of 1:10 or 1:100, and were stimulated with 1 µg/ml CpG plus 50 ng/ml IL-2. The AE was derived from 6 PB samples from CLL patients. The plasma and erythrocyte fractions from the 6 samples were mixed in a 1:1 ratio, and added to the wells at 1 µl/well. Results show that HS significantly increases the viability of the tumor cells over FBS in all conditions (Figure 7). The median value for each sample tested is shown. Increasing both HS and FBS to 20% leads to an increase in cell viability but the higher serum concentrations also inhibit proliferation. Co-culture with the HS-5 cell line reduces viability in the HS samples, but is necessary for proliferation. The best condition overall for viability and proliferation is 10% HS.

### Example 8 - Viability and Proliferation Using CLLPB +/- HS-5 Cells to Determine Best Cytokine Protocol using Table 2 conditions

The present experiment evaluates the effect of viability and proliferation of 24 cytokines combinations (Table 2) that cover different cell signaling. The cytokines used were: CpG (1 µg/ml), IL-2 (50 ng/ml), sCD40L (1 µg/ml), B cell activating factor (BAFF, 10 ng/ml), IgM+IgG (BCR, 10 µg/ml), IL-21 (Interleukin-21, 50 ng/ml). When included, stromal cells, HS-5 were used at a dilution of 1:100. The AE for these experiments was plasma and erythrocytes, obtained from PB of 3 patients with CLL. The AE fractions from each sample were mixed in equal parts prior to adding to the culture media. The media used contained the AE (1.6 %)plus human serum (HS) 10%. 20 CLL cryopreserved primary tumor samples were processed, and tested with each of the cytokine combinations. Both proliferation (X-axis) and viability (Y-axis) were tested as previously described for each cytokine combination. The numbers indicated in Table 2 correspond to each combination represented in Figures 8 and 9.

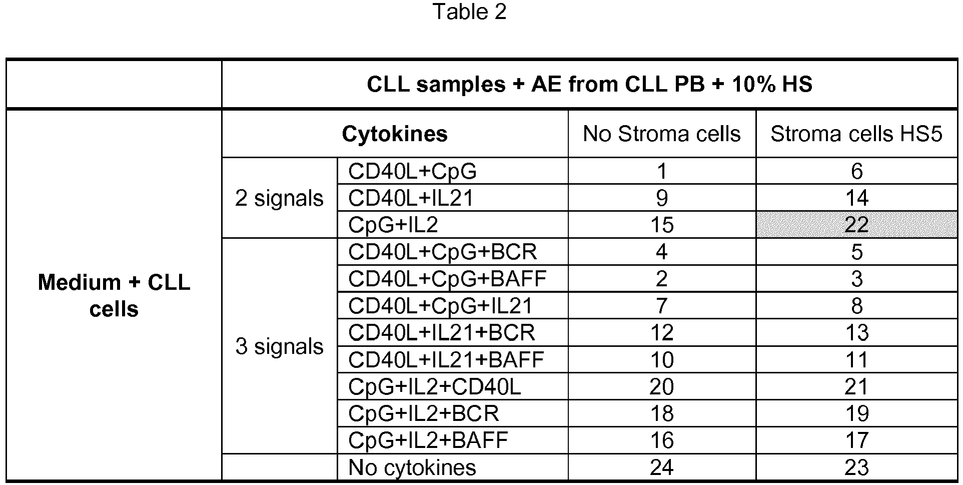

Each data point of the dot plot in Figure 8 corresponds to one specific cytokine combination indicated by the number. Figure 8 reflects the median values for all the samples for each combination, showing that the cytokine combination of CpG (1 µg/ml) plus IL2 (50 ng/ml) plus HS5 (1:100) together with CLL PB AE and HS 10% is the best condition, providing up to 60% viability and 40% proliferation at 96 h of incubation. This is combination 22 in the table and on figure 8.

Figure 9 groups all the previous 24 cytokine combinations by three different backbone stimulations, previously reported to improve to different extent CLL proliferation (X-axis) and viability (Y-axis). These correspond to sCD40L + CpG, sCD40L + IL-21 and CpG + IL-2. Figure 9, using the same combination numbers from Table 2, represents the median value of all the samples for the cytokine combination for each backbone stimulation. Figure 9 indicates that the cell signaling combination CpG + IL-2 is the best pathway to induce higher proliferation (20%) leaving a good viability (70%) compared to control in comparison with the other cell signaling pathways.

### Example 9 -Antiproliferative Activity of Ibrutinib and Idelalisib Tested on Primary CLL Cells in the Presence of AE

Cryopreserved Peripheral Blood (PB) mononuclear cells were provided from 15 CLL patients in need of treatment. The AE for these experiments was plasma and erythrocytes, obtained from PB of 3 patients with CLL. The AE fractions from each sample were mixed in equal parts prior to adding to the culture media. Cryopreserved CLL primary tumor cells to be tested were cultured at a ratio of 100:1 on confluent layers of HS-5 cell line in AIM-V (AIM-V®+ Albumax® (BSA 1X)) supplemented with 1.6% AE, 10% (vol/vol) Human Serum, 2% HEPES, 1% antibiotic, and 1% L-glutamine 200 mM. Cells were stimulated with the following pair cytokine combination: 1 µg/ml CpG ODN plus 50 ng/ml IL-2 for 96 hours at 37°C in humidified air containing 5% CO₂. CLL cell viability (assessed by Annexin V staining) and proliferation (assessed by the CFDA dye) is tested with 8 concentrations of Ibrutinib (N=15) and Idelalisib (N=21) that cover the range of activities across patients. Figure 10A-C shows the Dose Response (DR) curves from the samples where each grey line represents a different sample. The survival index of the B-Cells is displayed in the Y-axis starting at 100%, and the X-axis represents the Ibrutinib (Figure 10A) or Idelalisib (Figure 10B) drug concentration. The left panels in Figures 10A and B show the effect of each drug in Non-Proliferative (NP) Live Tumor Cells and the right panels show the effect of the drugs in Proliferative (PR) cell fraction. Figure 10A and B demonstrates that both Ibrutinib (A) and Idelalisib (B) have little effect on the NP fraction, suggesting a limited direct pro-apoptotic activity of the drugs. By contrast, a potent inhibition of the proliferation is observed in the low nM range blocking almost completely the proliferation (PR). Figure 10C shows the median values of both NP (grey line) and PR (dark line) cell subsets for Ibrutinib and Idelalisib and reflect the clear difference in the sensitivity of NP vs PR cells. These results reflect the necessity to recreate the *in vivo* microenvironment of CLL, here represented with the addition of the AE, stromal cells and cytokines to predict the effect of drugs such as Ibrutinib and Idelalisib.

### Example 10 - Viability and Absolute CD34 Blast Recovery in MDS samples with Different Test Conditions

AML and MDS are both associated with malignancies in multipotential hemopoietic stem cells that can be identified by their expression of CD34+. These cells can prove to be difficult to keep viable *ex vivo,* and have very low levels of proliferation. An appropriate source of serum, media or AE should be used to avoid CD34+ cell death and allow proliferation with specific cytokines. In these experiments, we tested cryopreserved primary cells (mononuclear) obtained from BM from a Myelodysplastic Syndrome (MDS) patient (Figure 11). Seven different conditions were tested plus a control, each for three times, 24h, 48h and 72h (each in triplicate all conditions except the control contained MyeloCultH5100 culture medium (Stemcell Technologies, Vancouver, Canada) containing 100 ng/ml Stem Cell Factor (Peprotech, London, United Kingdon) plus 50 ng/ml IL-3. Each number below shows a different component that was added to a sample set, and also corresponds with the numbers on figure 11, showing the results:
1. + 10% (vol/vol) of supernatant of HS5 cell line.
2. + 20% (vol/vol) of supernatant of HS5 cell line.
3. +1.6% AE (plasma and erythrocytes fraction) from Normal Bone Marrow (NBM).
4. +10% (vol/vol) Human Serum (HS).
5. + 20% (vol/vol) Human Serum, RPMI + 20% (vol/vol) Fetal Bovine Serum (FBS).
6. +high confluent of HS5 cell line plus 20% (vol/vol) Fetal Bovine Serum (FBS).
7. + low confluent of HS5 cell line plus 20% (vol/vol) Fetal Bovine Serum (FBS).

The Control (CON) was incubated in RPMI + 20% FBS. Viability was assessed by Annexin V and absolute CD34+ cell counts by our ExviTech® platform. For analysis, the antibodies CD45-APC (Clon HI30, Immunostep) and CD34-PerCP (Clon581, Biolegend) are used to identify the primary CD34+ cells. The upper panel in Figures 11 shows viability and the lower panel is absolute CD34+ blast cell counts. The different conditions, including the addition of AE, affects both parameters. Many of these conditions show a similar pattern with viability after cell culture being comparable to control, and the same for CD34+ numbers vs. baseline. However, interestingly for this particular MDS sample, the addition of the AE from only 1 NBM (number 3) and the co-culture with the HS5 stromal cell line (high and low confluence, numbers 6 and 7) decrease the CD34+ viability.

### Example 11 - Viability and Proliferation in 4 B-NHL Primary Samples with Different Cytokine Cocktails and AE from Normal BM

As shown for CLL samples, the addition of different cytokine combinations and/or AE could improve the viability or proliferation of B-Non Hodkin's Lymphoma (B-NHL) primary samples (Figures 12A and B). In this experiment, we tested both viability (Y-axis) and proliferation (X-axis) as previously described with Annexin V and CFDA at two times, 72h and 96h, for different conditions. Four cryopreserved B-NHL primary tumor samples were included: 2 follicular lymphoma (FL) and 2 Splenic Marginal Zone Lymphomas (SMZL). Fifteen different conditions were tested as shown in Table 3 to optimize for viability and proliferation. The numbers of each condition of Table 3 correspond to the samples in Figure 12. The AE used for this example consisted of plasma and erythrocytes fraction at a concentration of 1.6% (vol/vol).

**Table 3**

| | **CLL cells + AIM-V** | **No AE** | **AE (NMB)** |
|---|---|---|---|
| **Cytokine combinations + 10% HS** | No | 1 | 6 |
| | HS5 (1 :100) | 4 | |
| | HS5Sup | 5 | 7 |
| | CpG+CD40L | 8 | 9 |
| | CpG+CD40L+HS5 (1:100) | | 10 |
| | CpG+CD40L+HS5Sup | | 11 |
| | CpG+IL2 | 12 | 13 |
| | CpG+IL2+ HS5 (1:100) | | 14 |
| | CpG+IL2+ HS5Sup | | 15 |
| **+ 20% HS** | | 2 | |
| **+ 10% FBS** | | 3 | |

For analysis, the antibodies CD19-PE (clone HIB19, e-Bioscience), CD10-PE-Cya7(clone HI10a, Biolegend), CD45PO (clone HI30, Invitrogen) and CD20 APC (clone L27, Becton Dickinson) are used to identify the primary lymphoma cells. The median response for the 4 B-NHL samples is shown for each condition tested, where each data point of the dot plots represents one combination in Table 3. Results for the 72-hour incubation are shown in Figure 12A, and the 96-hour incubation is shown in Figure 12B, where the graphs show viability on the Y-axis and proliferation on the X-axis. Filled squares are combinations containing CD40L, open squares are combinations containing IL2, and circles do not contain cytokines. As showed for CLL, different conditions retain viability but a CpG + sCD40L (filled squares) is necessary to induce the most proliferation. The best overall condition was 96h incubation with CpG+sCD40L+HS 10% with (or without) the AE of the NBM (Figure 12B right two data points, 8 and 9). Both have viability and proliferation levels greater than 60%.

### Example 12 - Viability and Proliferation in MM Primary Samples

Bone marrow samples obtained from MM patients were evaluated to determine the best conditions for preserving viability and proliferation rates. Different sample treatments were analyzed to determine how they affect the viability and/or proliferation of the tumor cells. Additionally, each sample was treated differently prior to its use in the assay. The conditions are as follows:
A. Whole bone marrow from MM patient + FBS (20%)
B. Bone marrow mononuclear cells isolated from the MM sample by Ficoll Hypaque sedimentation + Autologous plasma (5, 10, 20 and 50%) and red blood cells.
C. Whole bone marrow with red blood cells lysed (by Ammonium Chloride solution & BulkLysis Cytognos) + Autologous plasma (20%)

Four types of culture medium were tested, including RPMI 1640, AIMV, DMEM and IMDM. The addition of two human stromal cell lines, HS27a and HS5, at different ratios -1:1, 1:10 and 1:100- were tested as well.

Patient whole BM was obtained and prepared as indicated above for each sample type. The Vybrant® CFDA SE Cell Tracer Kit (Molecular Probes) was used to measure cell proliferation as it has been disclosed in Example 3. After the CFDA protocol, all samples were resuspented in Culture Medium + Zell Shield (Labclinics) + L-Glu (Lonza) + 20% FBS. Condition A samples stayed as is, condition B samples had different concentrations of autologous MM plasma plus RBCs added and condition C samples received 20% autologous plasma. The final cell concentration ranged from 20 cells/µl to 70 cells/µl. Alternatively, the autologous plasma can be replaced with a pool of three MM plasmas.

The CFDA labeled plasmatic cells were dispensed into 96-well plates (60µl/well) and incubated at 37°C with 5% CO₂ during 72 or 96 hours. For analysis, MM cells (assessed by monoclonal antibodies) were tested for both viability (Annexin-V staining) and proliferation (CFDA).

After 96 hours of culture, the CFDA staining in conjunction with the immunophenotyping allowed for the analysis of both viability (Annexin-V negative) and proliferation (CFDA peaks) of the plasmatic cells, following the previously described methods using Vivia's ExviTech platform.

As the number of MM cells detected after 96 hours of incubation was too low in just a single well, a pooled well assay was performed, obtaining sample from 4 wells that have been treated in an identical manner. Thus, a significantly higher number of cells can be evaluated, increasing the reliability of the data. According to this, and based on the initial cell count, calculation of the sample volume was done for plating between 1000 and 4000 live pathological MM cells per well. During acquisition, sample from four wells was obtained for each treatment, thus obtaining a minimum of 4000 and a maximum of 16000 live plasmatic cells. The results of these experiments indicate that the use of patient's whole bone marrow plus 20% of autologous plasma, or a pool of at least 3 MM plasmas (Condition C from above) and the use of IMDM culture medium provided the best conditions to achieve better MM cells viability and higher proliferation rates (data not shown). Additionally, the presence of either of the two human stromal cell lines, HS27a and HS5, appeared to have a negative effect on both viability and proliferation.

A follow up experiment was conducted to analyze the effect of two cytokines, IL-6 (Interleukin-6) and CpG in this experimental setup. Table 4 shows the specific conditions assayed in each sample. Experimental procedures were as stated above. When cytokines were included (marked as X), they were added at the beginning of the incubation period.

**Table 4**

| No. | Sample | | Culture Media | Stromal Cells | MM Cell: Stromal cell Ratio | FBS (%) | Plasma (%) | IL-6 | CpG |
|---|---|---|---|---|---|---|---|---|---|
| 1 | B (Ficoll) | 1A | RPMI | no | | 20 | | | |
| | | 1B | AIM-V | HS27a | 1:100 | | 20 | X | X |
| 2 | B (Ficoll) | 2A | RPMI | no | | 20 | | | |
| | | 2B | AIM-V | HS27a | 1:100 | | 20 | X | X |
| 3 | B (Ficoll) | 2A | RPMI | no | | 20 | | | |
| | | 3B | AIM-V | HS27a | 1:100 | | 20 | X | X |
| 4 | C (Whole BM) | 4A | IMDM | HS27a | 1:10 | 13,75 | 6.25 | | |
| | | 4B | | | | | | X | |
| 5 | C (Whole BM) | 5A | IMDM | no | | 7,6 | 12,4 | | |
| | | 5B | | HS5 | 1:20 | | | | |
| 6 | C (Whole BM) | 6A | IMDM | HS27a | 1:10 | 11,1 | 9 | | |
| | | 6B | | | | | | X | |
| 7 | C (Whole BM) | 7A | IMDM | no | | 16,5 | 3,5 | | |
| | | 7B | | | | | | X | X |

The sample type refers to the different sample treatments outlined at the beginning of this example. All samples were assayed in duplicate (except sample 5). The numbers correspond to the samples in Figure 13. Results are shown for both the Non-Proliferative (NP) Live Tumor Cells (empty circles) and Proliferative (PR) cell fractions (grey circles).

The result indicates that the presence of cytokines does not provide any advantage in the viability of the cells nor in percentage of proliferation of the cells. Sample 1 even displayed higher levels of viability in the absence of IL-6 and CpG. Sample 7, which did not include stromal cells, displayed the highest level of viability and proliferation, which corresponds with previous results. Here again, cytokines did not have any effect on the outcome. It can be concluded that IL-6 and CpG do not appear to affect viability and proliferation of MM cells in *ex vivo* assays. The presence of autologous plasma, minus stromal cells and IL-6 offers the best conditions so far for MM cells. Other cytokines might have a different outcome.

### Example 13 - Antiproliferative Activity of Lenalidomide Tested on Primary MM Cells in the Presence of AE (plasma only)

Some drugs used to treat tumor cells preferentially target cells that are actively proliferating, acting as anti-proliferative agents. An example of this is the use of lenalidomide to treat patients diagnosed with MM. The efficacy of lenalidomide is difficult to assess in *ex vivo* assays against MM primary tumor cells, as these cells have very low levels of proliferation under the conditions that have traditionally been used. In this example, we tested BM samples from 10 patients diagnosed with MM in our *ex vivo* assay. AE (plasma only) was added to the culture medium to increase levels of proliferation and lenalidomide was tested across a concentration range of 0.03 to 30 µM.

Whole BM from 10 MM patients was cultured in IMDM medium supplemented with 1% antibiotic, 1% L-Glutamine plus an additional 20% of autologous plasma (AE), for 96 hours. Samples were processed and analyzed as outlined in Example 12. MM cell viability (Annexin V) and proliferation (CFDA) were tested against 4 concentrations of Lenalidomide.

Figure 14 shows results from four representative samples. The number of viable cells in both the non-proliferative (NP, grey) the proliferative (PR, black) populations for samples 1, 3, 6 and 7 are shown in Figure 14A. The presence of the AE induces a proliferating population in all of the samples. Dose Response (DR) curves from these four samples in response to lenalidomide (Figure 14B) show results for the NP MM cells (grey squares) and the PR MM cells (black circles). The survival index of the MM cells is displayed on the Y-axis, and the X-axis represents the lenalidomide drug concentration. This figure demonstrates that there is a clear difference in the sensitivity of lenalidomide between NP and PR MM cell populations. It also shows that there is a large level of interpatient variability. For sample 1 (Figure 14B upper left) lenalidomide has no effect on either PR or NP cells. The upper right graph shows that lenalidomide is very potent against the PR population for sample 3, working at a low concentration, but only eliminates about 60% of the PR cells. At a higher concentration, it also eliminates about 20% of the NP cells. For sample 6 (Figure 14B lower left) there is no effect on the PR population, but there is a small effect in the NP population. While in sample 7 (lower right) it can be seen that lenalidomide is very potent and effective against the PR cells, and at a higher concentration also effects the NP cells. These results show that the effectiveness of lenalidomide can vary greatly between patient samples in *ex vivo* assays, as it does between patients clinically. Also, it appears to have both anti-proliferative and cytotoxic properties, for some examples the anti-proliferative effect is more potent, occurring at a lower concentration that the cytotoxicity.

It can be concluded that the addition of the AE to the culture medium can change how cells react to a drug in an *ex vivo* setting. This may help to eventually correlate the *ex vivo* effect of drugs like lenalidomide to clinical outcomes specifically for each particular patient.

### BIBLIOGRAPHY

1. Pottier, C., Wheatherspoon, A., Roncarati, P., Longuespee, R., Herfs, M., Duray, A., Delvenne, P., and Quatresooz, P. (2015) The importance of the tumor microenvironment in the therapeutic management of cancer, Expert Rev Anticancer Ther 15, 943-954.
2. Sounni, N. E., and Noel, A. (2013) Targeting the tumor microenvironment for cancer therapy, Clin Chem 59, 85-93.
3. Buggins, A. G., Pepper, C., Patten, P. E., Hewamana, S., Gohil, S., Moorhead, J., Folarin, N., Yallop, D., Thomas, N. S., Mufti, G. J., Fegan, C., and Devereux, S. (2010) Interaction with vascular endothelium enhances survival in primary chronic lymphocytic leukemia cells via NF-kappaB activation and de novo gene transcription, Cancer Res 70, 7523-7533.
4. Ding, W., Nowakowski, G. S., Knox, T. R., Boysen, J. C., Maas, M. L., Schwager, S. M., Wu, W., Wellik, L. E., Dietz, A. B., Ghosh, A. K., Secreto, C. R., Medina, K. L., Shanafelt, T. D., Zent, C. S., Call, T. G., and Kay, N. E. (2009) Bi-directional activation between mesenchymal stem cells and CLL B-cells: implication for CLL disease progression, Br J Haematol 147, 471-483.
5. Kurtova, A. V., Balakrishnan, K., Chen, R., Ding, W., Schnabl, S., Quiroga, M. P., Sivina, M., Wierda, W. G., Estrov, Z., Keating, M. J., Shehata, M., Jager, U., Gandhi, V., Kay, N. E., Plunkett, W., and Burger, J. A. (2009) Diverse marrow stromal cells protect CLL cells from spontaneous and drug-induced apoptosis: development of a reliable and reproducible system to assess stromal cell adhesion-mediated drug resistance, Blood 114, 4441-4450.
6. Gill, B. J., and West, J. L. (2014) Modeling the tumor extracellular matrix: Tissue engineering tools repurposed towards new frontiers in cancer biology, J Biomech 47, 1969-1978.
7. Mallikarjun Sundaram, P. M., Misti Jain, Saravanan Thiagarajan, Dency Pinto, Padhma Radhakrishnan. (2014) ECM Composition, Tumor Microenvironment Platform and Methods Thereof. (UDPTO, Ed.), Mitra Biotech, India.
8. Mongini, P. K., Gupta, R., Boyle, E., Nieto, J., Lee, H., Stein, J., Bandovic, J., Stankovic, T., Barrientos, J., Kolitz, J. E., Allen, S. L., Rai, K., Chu, C. C., and Chiorazzi, N. (2015) TLR-9 and IL-15 Synergy Promotes the In Vitro Clonal Expansion of Chronic Lymphocytic Leukemia B Cells, J Immunol 195, 901-923.
9. Pascutti, M. F., Jak, M., Tromp, J. M., Derks, I. A., Remmerswaal, E. B., Thijssen, R., van Attekum, M. H., van Bochove, G. G., Luijks, D. M., Pals, S. T., van Lier, R. A., Kater, A. P., van Oers, M. H., and Eldering, E. (2013) IL-21 and CD40L signals from autologous T cells can induce antigen-independent proliferation of CLL cells, Blood 122, 3010-3019.
10. Nabhan, C., and Rosen, S. T. (2014) Chronic lymphocytic leukemia: a clinical review, JAMA 312, 2265-2276.
11. Mittal, A. K., Chaturvedi, N. K., Rai, K. J., Gilling-Cutucache, C. E., Nordgren, T. M., Moragues, M., Lu, R., Opavsky, R., Bociek, G. R., Weisenburger, D. D., Iqbal, J., and Joshi, S. S. (2014) Chronic lymphocytic leukemia cells in a lymph node microenvironment depict molecular signature associated with an aggressive disease, Mol Med 20, 290-301.
12. Burger, J. A., and Buggy, J. J. (2013) Bruton tyrosine kinase inhibitor ibrutinib (PCI-32765), Leuk Lymphoma 54, 2385-2391.
13. Chung, C., and Lee, R. (2014) Ibrutinib, obinutuzumab, idelalisib, and beyond: review of novel and evolving therapies for chronic lymphocytic leukemia, Pharmacotherapy 34, 1298-1316.
14. Purroy, N., Abrisqueta, P., Carabia, J., Carpio, C., Palacio, C., Bosch, F., and Crespo, M. (2015) Co-culture of primary CLL cells with bone marrow mesenchymal cells, CD40 ligand and CpG ODN promotes proliferation of chemoresistant CLL cells phenotypically comparable to those proliferating in vivo, Oncotarget 6, 7632-7643.
15. Bennett, T. A., Montesinos, P., Moscardo, F., Martinez-Cuadron, D., Martinez, J., Sierra, J., Garcia, R., de Oteyza, J. P., Fernandez, P., Serrano, J., Fernandez, A., Herrera, P., Gonzalez, A., Bethancourt, C., Rodriguez-Macias, G., Alonso, A., Vera, J. A., Navas, B., Lavilla, E., Lopez, J. A., Jimenez, S., Simiele, A., Vidriales, B., Gonzalez, B. J., Burgaleta, C., Hernandez Rivas, J. A., Mascunano, R. C., Bautista, G., Perez Simon, J. A., Fuente Ade, L., Rayon, C., Troconiz, I. F., Janda, A., Bosanquet, A. G., Hernandez-Campo, P., Primo, D., Lopez, R., Liebana, B., Rojas, J. L., Gorrochategui, J., Sanz, M. A., and Ballesteros, J. (2014) Pharmacological profiles of acute myeloid leukemia treatments in patient samples by automated flow cytometry: a bridge to individualized medicine, Clin Lymphoma Myeloma Leuk 14, 305-318.
16. Ghamlouch, H., Ouled-Haddou, H., Damaj, G., Royer, B., Gubler, B., and Marolleau, J. P. (2013) A combination of cytokines rescues highly purified leukemic CLL B-cells from spontaneous apoptosis in vitro, PLoS One 8, e60370.
17. Brinton, L. T., Sloane, H. S., Kester, M., and Kelly, K. A. (2015) Formation and role of exosomes in cancer, Cell Mol Life Sci 72, 659-671.
18. Benien, P., and Swami, A. (2014) 3D tumor models: history, advances and future perspectives, Future Oncol 10, 1311-1327.
19. Fennema, E., Rivron, N., Rouwkema, J., van Blitterswijk, C., and de Boer, J. (2013) Spheroid culture as a tool for creating 3D complex tissues, Trends Biotechnol 31, 108-115.
20. Nam, K. H., Smith, A. S., Lone, S., Kwon, S., and Kim, D. H. (2015) Biomimetic 3D Tissue Models for Advanced High-Throughput Drug Screening, J Lab Autom 20, 201-215.
21. Kucerova, L., and Skolekova, S. (2013) Tumor microenvironment and the role of mesenchymal stromal cells, Neoplasma 60, 1-10.
22. Ghia, P., Circosta, P., Scielzo, C., Vallario, A., Camporeale, A., Granziero, L., and Caligaris-Cappio, F. (2005) Differential effects of CLL cell survival exerted by different microenvironmental elements, Curr Top Microbiol Immunol 294, 135-45.
23. Roecklein, B. A., and Torok-Storb, B. (1995) Functionally distinct human marrow stromal cell lines immortalized by transduction with the human papilloma virus E6/E7 genes, Blood 85, 997-1005.

## Claims

1. An *ex vivo* method for testing cellular responsiveness of primary tumor cell populations to a drug or combination of drugs that comprises:
i) submit a whole sample from a subject selected from: peripheral blood (PB), or bone marrow (BN), or lymph node (LN) to a separation process to isolate an Artificial Environment (AE) consisting of a plasma fraction, an erythrocyte fraction or a combination thereof, free from leukocytes,
ii) mix the leukocyte-free AE obtained in the previous step with a primary tumor cell population,
iii) add to the mixture of step ii) at least one drug to be tested,
iv) incubate the mixture obtained in step iii) during from 2 hours to 14 days to allow the drug tested to exert any activity it might have on the primary tumor cell population,
v) assess the viability and/or proliferation of the primary tumor cell population in the presence or absence of the drug tested,
vi) produce comparative data on viability and/or on proliferation of the primary tumor cell population between the assessment made in presence and in absence of the drug tested and relate the data obtained to values indicative of drug activity for reducing/increasing viability and/or proliferation of the primary tumor cell population.

2. The *ex vivo* method of claim 1, wherein the combination of the plasma fraction and the erythrocyte fraction is at 1:1 ratio.

3. The *ex vivo* method of any of claims 1 to 2, wherein the leukocyte-free AE is obtained from a healthy donor, from a donor who has suffered or suffers from cancer, from several donors and pooled together before being mixed up with the primary tumor cell population and/or the drug to be tested, from a donor or donors which suffer from the same disease as the patient from which the primary tumor cell population is obtained, from a donor or donors which suffer from a different disease as the patient who provides the primary tumor cell population, from healthy donors or from the same subject providing the primary tumor cell population.

4. The *ex vivo* method of any of claims 1 to 3, wherein the incubation mixture also includes at least one soluble factor and/or at least one additional cell population of a cell type found in an *in vivo* tumor microenvironment cell population.

5. The *ex vivo* method of claim 4, wherein the at least one soluble factor is a non-cellular component of the tumor microenvironment selected from: tumor derived exosomes, signaling molecules, growth factors, chemokines, cytokines, interleukins and any soluble molecule derived from tumor or non-tumor cells.

6. The *ex vivo* method of claim 5, wherein the at least one soluble factor is selected from: Interleukin IL-2, IL-4, IL-6, IL-8, IL-10, IL-13, IL-15, IL-21, soluble cluster of differentiation (sCD) 40 ligand, Immunoglobulin (Ig) M, CpG, short synthetic single-stranded DNA molecules containing non-methylated CpG dinucleotides motifs, B cell activating factor (BAFF) and Proliferation Inducing Ligand (APRIL).

7. The *ex vivo* method of claim 4, wherein the at least one additional cell population is selected from: fibroblastic reticular cells, nurse-like cells, mesenchymal stem cells, follicular dendritic cells, endothelial cells, pericytes, and cell lines derived from primary stromal cells.

8. The *ex vivo* method of claim 7, wherein cell lines derived from primary stromal cells are selected from: HS-5, HS27a, UE6E7T-2, NK-Tert, HMEC-1, KUSA-H1, M210B4, ST-2, and KUM-4.

9. The *ex vivo* method of any of claims 4 to 8, wherein the at least one soluble factor and/or additional cell population is from other species than human.

10. The *ex vivo* method of any of claims 1 to 9, wherein the primary tumor cell population has been freshly extracted before their use or have been previously cryopreserved and thawed, together with the AE, before use.

11. The *ex vivo* method of any of claims 1 to 9, wherein the primary tumor cell population has been extracted from a patient who has not received a prior treatment for a cancer, or has been extracted from a cancer patient who has received one or more previous treatments for said cancer, or the primary tumor cell population has been extracted from a patient with Minimal Residual Disease (MRD).

12. The *ex vivo* method of any of claims 1 to 11, wherein the primary tumor cell population is obtained from a solid tumor selected from a list which comprises: ovarian cancer, rectal cancer, cancer of the anal region, stomach cancer, testicular cancer, uterine cancer, colon cancer, rectal cancer, renal-cell carcinoma, liver cancer, non-small cell carcinoma of the lung, cancer of the small intestine, cancer of the esophagus, melanoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, carcinoma of the fallopian tubes, brain stem glioma, pituitary adenoma, Kaposi's sarcoma, epidermoid cancer, squamous cell cancer, carcinoma of the endometrium, carcinoma of the cervix, carcinoma of the vagina, carcinoma of the vulva, sarcoma of soft tissue, cancer of the urethra, cancer of the penis, cancer of the bladder, cancer of the kidney or ureter, carcinoma of the renal pelvis, neoplasm of the central nervous system (CNS), primary CNS lymphoma, tumor angiogenesis, spinal axis tumor, metastatic lesions of said cancers, or combinations thereof.

13. The *ex vivo* method of any of claims 11 or 12, wherein the method comprises 3D cell culture constructs built to mimic the microenvironment architecture of solid tumors, selected from: spheroids, extracellular matrix gels, synthetic scaffolds, rotary cell culture systems, or on low/non-adherent culture plastics.

14. The *ex vivo* method of any of claims 1 to 13, wherein the primary tumor cell population is obtained from a hematological disorder selected from Hodgkin's lymphoma, Non-Hodgkin's lymphoma, B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia, chronic lymphocytic leukemia, acute myeloid leukemia, chronic myeloid leukemia, myelodysplastic syndrome, multiple myeloma, or acute lymphocytic leukemia, autoimmune diseases, blood coagulation disorders or anemia.

15. The *ex vivo* method of any of claims 1 to 14, wherein the at least one drug tested comprises at least one therapeutic agent selected from a list which includes: a chemotherapeutic drug, a targeted anti-cancer therapy drug, an oncolytic drug, a cytotoxic agent, an immune-based therapy drug, a cytokine, drugs targeting the microenvironment, an activator of a costimulatory molecule, an inhibitor of an inhibitory molecule, or an immunotherapy agent.

16. The *ex vivo* method of any of claims 1 to 15, wherein the assessment of the cellular response of the primary tumor cell population includes measuring at least one of the following parameters: viability, proliferation, apoptosis, cell depletion, changes in surface marker expression, changes in cytokine/chemokine production.

17. The *ex vivo* method of any of claims 1 to 16, for use to determine the effectiveness of a prescribed treatment to a subject after treatment has been initiated.

18. The *ex vivo* method of claim 17, wherein the determination of the effectiveness is at least seven days after treatment has been initiated.

## Patentansprüche

1. *Ex-vivo*-Verfahren zur Untersuchung der zellulären Ansprechbarkeit von primären Tumorzellpopulationen auf ein Arzneimittel oder eine Kombination aus Arzneimitteln, das Folgendes umfasst:
i) Unterziehen einer Gesamtprobe eines Subjekts, ausgewählt aus: peripherem Blut (PB) oder Knochenmark (BN) oder Lymphknoten (LN), einem Trennprozess, um eine künstliche Umgebung (AE) zu isolieren, die aus einer Plasmafraktion, einer Erythrozytenfraktion oder einer Kombination davon besteht und frei von Leukozyten ist,
ii) Mischen der leukozytenfreien, im vorherigen Schritt erhaltenen AE mit einer primären Tumorzellpopulation,
iii) Zugeben von mindestens einem zu untersuchenden Arzneimittel zu der Mischung aus Schritt ii),
iv) Inkubieren des in Schritt iii) erhaltenen Gemischs für 2 Stunden bis 14 Tage, um dem Arzneimittel die Ausübung einer Aktivität, die es auf die primäre Tumorzellpopulation haben könnte, zu ermöglichen,
v) Beurteilen der Lebensfähigkeit und/oder der Proliferation der primären Tumorzellpopulation in Anwesenheit oder Abwesenheit des untersuchten Arzneimittels,
vi) Erzeugen von Vergleichsdaten zur Lebensfähigkeit und/oder Proliferation der primären Tumorzellpopulation zwischen der in Anwesenheit und in Abwesenheit des untersuchten Arzneimittels durchgeführten Beurteilung und Zuordnen der erhaltenen Daten zu Werten, die bezeichnend für eine Arzneimittelaktivität im Sinne einer Verringerung/Erhöhung der Lebensfähigkeit und/oder der Proliferation der primären Tumorzellpopulation sind.

2. *Ex-vivo*-Verfahren nach Anspruch 1, wobei die Kombination der Plasmafraktion und der Erythrozytenfraktion im Verhältnis von 1:1 vorliegt.

3. *Ex-vivo*-Verfahren nach einem der Ansprüche 1 bis 2, wobei die leukozytenfreie AE gewonnen wird aus einem gesunden Spender, aus einem Spender, der an Krebs gelitten hat oder leidet, aus mehreren Spendern, deren AE vereinigt werden, bevor sie mit der primären Tumorzellpopulation und/oder dem zu untersuchenden Arzneimittel gemischt werden, aus einem Spender oder aus Spendern, die an derselben Erkrankung leiden wie der Patient, aus dem die primären Tumorzellpopulation erhalten wird, aus einem Spender oder aus Spendern, die an einer anderen Erkrankung leiden als der Patient, der die primäre Tumorzellpopulation bereitstellt, aus gesunden Spendern oder aus demselben Subjekt, der die primäre Tumorzellpopulation bereitstellt.

4. *Ex-vivo*-Verfahren nach einem der Ansprüche 1 bis 3, wobei das Inkubationsgemisch ebenfalls mindestens einen löslichen Faktor und/oder mindestens eine zusätzliche Zellpopulation eines Zelltyps beinhaltet, der *in vivo* in einer Zellpopulation der Tumormikroumgebung vorkommt.

5. *Ex-vivo*-Verfahren nach Anspruch 4, wobei der mindestens eine lösliche Faktor eine nicht-zelluläre Komponente der Tumormikroumgebung ist, die ausgewählt ist aus: tumorabgeleiteten Exosomen, Signalmolekülen, Wachstumsfaktoren, Chemokinen, Zytokinen, Interleukinen und einem löslichen Molekül, das von Tumor- oder Nicht-Tumorzellen abgeleitet ist.

6. *Ex-vivo*-Verfahren nach Anspruch 5, wobei der mindestens eine lösliche Faktor ausgewählt ist aus: Interleukin IL-2, IL-4, IL-6, IL-8, IL-10, IL-13, IL-15, IL-21, Ligand des löslichen Differenzierungsclusters (sCD) 40, Immunglobulin (Ig) M, CpG, kurzen synthetischen Einzelstrang-DNA-Molekülen, die nichtmethylierte CpG-Dinukleotidmotive enthalten, B-Zell-Aktivierungsfaktor (AFF) und proliferationinduzierendem Ligand (APRIL).

7. *Ex-vivo*-Verfahren nach Anspruch 4, wobei die mindestens eine zusätzliche Zellpopulation ausgewählt ist aus: fibroblastischen Retikulumzellen, Ammenzellen, mesenchymalen Stammzellen, follikulären dendritischen Zellen, Endothelzellen, Perizyten und von primären Bindegewebszellen abgeleiteten Zelllinien.

8. *Ex-vivo*-Verfahren nach Anspruch 7, wobei die von primären Bindegewebszellen abgeleiteten Zelllinien ausgewählt sind aus: HS-5, HS27a, UE6E7T-2, NK-Tert, HMEC-1, KUSA-H1, M210B4, ST-2 und KUM-4.

9. *Ex-vivo*-Verfahren nach einem der Ansprüche 4 bis 8, wobei der mindestens eine lösliche Faktor und/oder die mindestens eine zusätzliche Zellpopulation aus einer vom Menschen verschiedenen Spezies stammt.

10. *Ex-vivo*-Verfahren nach einem der Ansprüche 1 bis 9, wobei die primäre Tumorzellpopulation vor ihrer Verwendung frisch entnommen wurde oder zuvor kryokonserviert und vor Verwendung zusammen mit der AE aufgetaut wurde.

11. *Ex-vivo*-Verfahren nach einem der Ansprüche 1 bis 9, wobei die primäre Tumorzellpopulation aus einem Patienten entnommen wurde, der keine frühere Behandlung für einen Krebs erhalten hatte, oder aus einem Krebspatienten entnommen wurde, der eine oder mehrere frühere Behandlungen für diesen Krebs erhalten hatte, oder die primäre Tumorzellpopulation aus einem Patienten mit einer minimalen Resterkrankung (MRD) entnommen wurde.

12. *Ex-vivo*-Verfahren nach einem der Ansprüche 1 bis 11, wobei die primäre Tumorzellpopulation aus einem soliden Tumor gewonnen wird, der aus einer Liste ausgewählt ist, die Folgendes umfasst: Eierstockkrebs, Enddarmkrebs, Krebserkrankung der Afterregion, Magenkrebs, Hodenkrebs, Gebärmutterkrebs, Darmkrebs, Enddarmkrebs, Nierenzellkarzinom, Leberkrebs, nicht-kleinzelligem Lungenkarzinom, Dünndarmkrebs, Speiseröhrenkrebs, Melanom, Krebserkrankungen des Hormonsystems, Schilddrüsenkrebs, Nebenschilddrüsenkrebs, Nebennierenkrebs, Knochenkrebs, Bauchspeicheldrüsenkrebs, Hautkrebs, Kopf- oder Halskrebs, kutanem oder intraokulärem malignem Melanom, Gebärmutterkrebs, Eileiterkarzinom, Hirnstammgliom, Hypophysenadenom, Kaposi-Sarkom, Epidermoidkrebs, Plattenepithelkarzinom, Endometriumkarzinom, Zervixkarzinom, Vaginalkarzinom, Vulvakarzinom, Weichteilsarkom, Harnröhrenkrebs, Peniskrebs, Blasenkrebs, Nieren- oder Harnleiterkrebs, Nierenbeckenkarzinom, Neoplasma des zentralen Nervensystems (ZNS), primäres ZNS-Lymphom, Tumorangiogenese, Wribelsäulentumor, metastatischen Läsionen dieser Krebserkrankungen und Kombinationen davon.

13. *Ex-vivo*-Verfahren nach einem der Ansprüche 11 oder 12, wobei das Verfahren 3D-Zellkulturkonstruktionen umfasst, die zur Nachahmung der Mikroumgebungsarchitektur solider Tumoren erzeugt werden und ausgewählt sind aus: Sphäroiden, extrazellulären Matrixgelen, synthetischen Gerüsten, rotierenden Zellkultursystemen oder auf wenig-/nicht-haftenden Kulturkunststoffen.

14. *Ex-vivo*-Verfahren nach einem der Ansprüche 1 bis 13, wobei die primäre Tumorzellpopulation aus einer hämtologischen Störung gewonnen wird, ausgewählt aus Hodgkin-Lymphom, Non-Hodgkin-Lymphom, B-Zell-Lymphom, diffusem großzelligem B-Zell-Lymphom, follikulärem Lymphom, Mantelzelllymphom, Marginalzonen-B-Zell, Burkitt-Lymphom, lymphoplasmozytischem Lymphom, Haarzellenleukämie, chronischer lymphozytischer Leukämie, akuter myeloischer Leukämie, chronischer myeloischer Leukämie, myelodysplastischem Syndrom, multiplen Myelom oder akuter lymphozytischer Leukämie, Autoimmunkrankheiten, Blutgerinnungsstörungen oder Anämie.

15. *Ex-vivo*-Verfahren nach einem der Ansprüche 1 bis 14, wobei das mindestens eine untersuchte Arzneimittel mindestens ein therapeutisches Mittel umfasst, das aus einer Liste ausgewählt ist, die Folgendes beinhaltet: ein Chemotherapeutikum, ein gezieltes Krebstherapeutikum, ein onkolytisches Arzneimittel, ein zytotoxisches Mittel, ein immunbasiertes Therapeutikum, ein Zytokin, Arzneimittel, die auf die Mikroumgebung gerichtet sind, ein Aktivator eines kostimulatorischen Moleküls, ein Inhibitor eines inhibitorischen Moleküls oder ein Immuntherapeutikum.

16. *Ex-vivo*-Verfahren nach einem der Ansprüche 1 bis 15, wobei die Beurteilung des zellulären Ansprechens der primären Tumorzellpopulation das Messen mindestens eines der folgenden Parameter beinhaltet: Lebensfähigkeit, Proliferation, Apoptose, Zellabreicherung, Veränderung der Expression von Oberflächenmarkern, Veränderungen der Zytokin-/Chemokin-Produktion.

17. *Ex-vivo*-Verfahren nach einem der Ansprüche 1 bis 16 zur Verwendung bei der Feststellung der Wirksamkeit einer verschriebenen Behandlung nach Einleiten der Behandlung bei einem Subjekt.

18. *Ex-vivo*-Verfahren nach Anspruch 17, wobei die Bestimmung der Wirksamkeit mindestens sieben Tage nach der Einleitung der Behandlung erfolgt.

## Revendications

1. Une méthode *ex vivo* de test de la réponse cellulaire de populations de cellules d'une tumeur primitive à un médicament ou une combinaison de médicaments comprenant les étapes suivantes :
i) soumettre un échantillon entier d'un sujet choisi parmi le sang périphérique (SP), la moelle osseuse (MO) ou un ganglion lymphatique (GL) à un processus de séparation afin d'isoler un milieu artificiel (MA) consistant en une fraction plasmatique, une fraction érythrocytaire ou une combinaison de celles-ci, exempt de leucocytes,
ii) mélanger le MA exempt de leucocytes obtenu à l'étape précédente avec une population de cellules d'une tumeur primitive,
iii) ajouter au mélange de l'étape ii) au moins un médicament à tester,
iv) faire incuber le mélange obtenu à l'étape iii) entre 2 heures et 14 jours afin de permettre au médicament testé d'exercer une éventuelle activité sur la population de cellules d'une tumeur primitive,
v) évaluer la viabilité et/ou la prolifération de la population de cellules d'une tumeur primitive en présence ou en l'absence du médicament testé,
vi) générer des données comparatives sur la viabilité et/ou la prolifération de la population de cellules d'une tumeur primitive entre l'évaluation effectuée en présence et en l'absence du médicament testé et associer les données obtenues aux valeurs indicatives de l'activité du médicament de réduction/d'augmentation de la viabilité et/ou la prolifération de la population de cellules d'une tumeur primitive.

2. La méthode *ex vivo* selon la revendication 1, dans laquelle la combinaison de la fraction plasmatique et de la fraction érythrocytaire correspond au rapport 1:1.

3. La méthode *ex vivo* selon l'une des revendications 1 et 2, dans laquelle le MA exempt de leucocytes est obtenu auprès d'un donneur sain, auprès d'un donneur qui a été atteint ou qui est atteint d'un cancer, auprès de plusieurs donneurs, les MA étant réunis avant d'être mélangés avec la population de cellules d'une tumeur primitive et/ou le médicament à tester, auprès d'un ou de plusieurs donneurs atteints de la même maladie que le patient à partir duquel la population de cellules d'une tumeur primitive est obtenue, auprès d'un ou de plusieurs donneurs atteints d'une maladie différente de celle du patient fournissant la population de cellules d'une tumeur primitive, auprès de donneurs sains, ou auprès du même sujet ayant fourni la population de cellules d'une tumeur primitive.

4. La méthode *ex vivo* selon l'une des revendications 1 à 3, dans laquelle le mélange d'incubation comprend également au moins un facteur soluble et/ou au moins une population de cellules supplémentaire d'un type de cellule trouvé dans une population de cellules d'un microenvironnement tumoral *in vivo.*

5. La méthode *ex vivo* selon la revendication 4, dans laquelle ledit au moins un facteur soluble est un composant non cellulaire du microenvironnement tumoral choisi parmi : des exosomes dérivés d'une tumeur, des molécules de signalisation, des facteurs de croissance, des chimiokines, des cytokines, des interleukines et toute molécule soluble dérivée de cellules tumorales ou non tumorales.

6. La méthode *ex vivo* selon la revendication 5, dans laquelle ledit au moins un facteur soluble est choisi parmi : les interleukines IL-2, IL-4, IL-6, IL-8, IL-10, IL-13, IL-15, IL-21, le ligand du cluster de différenciation soluble (CDs) 40, les immunoglobulines M (Ig), le CpG, des molécules d'ADN synthétique simple brin courtes contenant des motifs de dinucléotides CpG non méthylés, le facteur d'activation des lymphocytes B (BAFF) et le ligand inducteur de prolifération (APRIL).

7. La méthode *ex vivo* selon la revendication 4, dans laquelle ladite au moins une population de cellules supplémentaire est choisie parmi : les cellules réticulaires fibroblastiques, les cellules nurse-like, les cellules souches mésenchymateuses, les cellules dendritiques folliculaires, les cellules endothéliales, les péricytes, et les lignées cellulaires dérivées de cellules stromales primaires.

8. La méthode *ex vivo* selon la revendication 7, dans laquelle les lignées cellulaires dérivées de cellules stromales primaires sont choisies parmi : HS-5, HS27a, UE6E7T-2, NK-Tert, HMEC-1, KUSA-H1, M210B4, ST-2, et KUM-4.

9. La méthode *ex vivo* selon l'une des revendications 4 à 8, dans laquelle ledit au moins un facteur soluble et/ou ladite au moins une population de cellules supplémentaire proviennent d'autres espèces que l'espèce humaine.

10. La méthode *ex vivo* selon l'une des revendications 1 à 9, dans laquelle la population de cellules d'une tumeur primitive a été fraîchement extraite avant d'être utilisée ou a été préalablement cryoconservée et décongelée, avec le MA, avant d'être utilisée.

11. La méthode *ex vivo* selon l'une des revendications 1 à 9, dans laquelle la population de cellules d'une tumeur primitive a été extraite d'un patient qui n'a pas reçu de traitement préalable contre le cancer, ou a été extraite d'un patient atteint d'un cancer qui a reçu un ou plusieurs traitements contre ledit cancer, ou la population de cellules d'une tumeur primitive a été extraite d'un patient atteint de la maladie résiduelle minime (MRM).

12. La méthode *ex vivo* selon l'une des revendications 1 à 11, dans laquelle la population de cellules d'une tumeur primitive est obtenue à partir d'une tumeur solide choisie dans une liste comprenant : le cancer de l'ovaire, le cancer du rectum, le cancer de l'anus, le cancer de l'estomac, le cancer du testicule, le cancer de l'utérus, le cancer du côlon, le carcinome à cellules rénales, le cancer du foie, le cancer bronchique non à petites cellules, le cancer de l'intestin grêle, le cancer de l'œsophage, le mélanome, le cancer du système endocrinien, le cancer de la glande thyroïde, le cancer de la parathyroïde, le cancer de la glande surrénale, le cancer des os, le cancer du pancréas, le cancer de la peau, le cancer de la tête ou du cou, le mélanome cutané ou intraoculaire, l'adénocarcinome de la trompe de Fallope, le gliome du tronc cérébral, l'adénome hypophysaire, la maladie de Kaposi, le carcinome épidermoïde, le carcinome cellulaire squameux, le carcinome endométrial, le carcinome cervical, le cancer du vagin, le cancer de la vulve, le sarcome des tissus mous, le cancer de l'urètre, le cancer du pénis, le cancer de la vessie, le cancer du rein ou de l'uretère, le cancer du bassinet du rein, le cancer du système nerveux central (SNC), le lymphone primitif du SNC, l'angiogenèse tumorale, la tumeur de l'axe spinal, les lésions métastatiques desdits cancers, ou une combinaison de ces derniers.

13. La méthode *ex vivo* selon l'une des revendications 11 ou 12, dans laquelle la méthode comprend des structures de culture cellulaire en 3 dimensions construites afin de reproduire l'architecture microenvironnementale de tumeurs solides, choisies parmi : des sphéroïdes, des gels de matrice extracellulaire, des échafaudages synthétiques, des systèmes de cultures cellulaires rotatives, ou des matières plastiques sur culture non adhérente/faiblement adhérente.

14. La méthode *ex vivo* selon l'une des revendications 1 à 13, dans laquelle la population de cellules d'une tumeur primitive est obtenue à partir d'un trouble hématologique choisi parmi le lymphome de Hodgkin, le lymphone non hodgkinien, le lymphone à cellules B, le lymphone diffus à grandes cellules B, le lymphome folliculaire, le lymphome des cellules du manteau, le lymphome à cellules de la zone marginale, le lymphome de Burkitt, le lymphome lymphoplasmocytaire, la leucémie à tricholeucocytes, la leucémie lymphoïde chronique, la leucémie aigüe myéloïde, la leucémie myéloïde chronique, le syndrome myélodysplasique, le myélome multiple, la leucémie aiguë lymphoblastique, les maladies auto-immunes, les troubles de la coagulation ou l'anémie.

15. La méthode *ex vivo* selon l'une des revendications 1 à 14, dans laquelle ledit au moins un médicament testé comprend au moins un agent thérapeutique choisi dans une liste comprenant : un agent chimiothérapeutique, un médicament de thérapie ciblée anticancéreuse, un médicament de thérapie oncolytique, un agent cytotoxique, un médicament d'immunothérapie, des médicaments ciblant le microenvironnement, un activateur de molécules de costimulation, un inhibiteur de molécules inhibitrices, ou un agent d'immunothérapie.

16. La méthode *ex vivo* selon l'une des revendications 1 à 15, dans laquelle l'évaluation de la réponse cellulaire de la population de cellules d'une tumeur primitive comprend la mesure d'au moins l'un des paramètres suivants : la viabilité, la prolifération, l'apoptose, la déplétion des cellules, les changements de l'expression du marqueur de surface, les changements de la production de cytokines/chimiokines.

17. La méthode *ex vivo* selon l'une des revendications 1 à 16, devant être utilisée afin de déterminer l'efficacité d'un traitement prescrit à un sujet après le début du traitement.

18. La méthode *ex vivo* selon la revendication 17, dans laquelle l'efficacité est déterminée au moins sept jours après le début du traitement.
